Europäisches Patentamt
European Patent Office
Office européen des brevets

(19)

(11) Veröffentlichungsnummer: **0 000 710**
**A1**

(12)

# EUROPÄISCHE PATENTANMELDUNG

(21) Anmeldenummer: 78100426.2

(22) Anmeldetag: 18.07.78

(51) Int. Cl.$^2$: C 07 C 177/00

(30) Priorität: 26.07.77 DE 2733680

(43) Veröffentlichungstag der Anmeldung:
21.02.79 Patentblatt 79/4

(84) Benannte Vertragsstaaten:
BE CH DE FR GB LU NL


(71) Anmelder: Bayer Aktiengesellschaft
Zentralbereich Patente,Marken und Lizenzen Bayerwerk
D-5090 Leverkusen 1(DE)

(72) Erfinder: Lieb, Folker, Dr.
Alfred-Kubin-Strasse 1
D-5090 Leverkusen 1(DE)

(72) Erfinder: Busse, Wolf-Dieter, Dr.
Pahlkestrasse 65
D-5600 Wuppertal 1(DE)

(72) Erfinder: Oediger, Hermann, Dr.
Roggendorfstrasse 51
D-5000 Köln 80(DE)

(72) Erfinder: Sitt, Rüdiger, Dr.
Claudiusweg 9
D-5600 Wuppertal 1(DE)

(72) Erfinder: Eiter, Karl, Prof.Dr.
An der Ruthen 5
D-5000 Koeln 80(DE)

(72) Erfinder: Hebenbrock, Klaus-Friedrich, Dr.
Schulstrasse 9a
D-5068 Odenthal(DE)


(54) **Prostaglandin-Analoga, Verfahren zu deren Herstellung und deren Verwendung als Arzneimittel.**


(57) Die vorliegende Erfindung betrifft Prostaglandin - Analoga der allgemeinen Formel

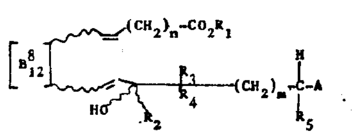

in welcher

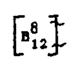 für die Teilstrukturen

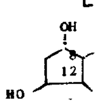 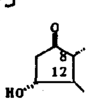 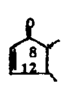 oder 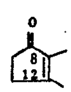

steht, sowie ein Verfahren zu deren Herstellung worin man ein Aldehyd der allgemeinen Formel

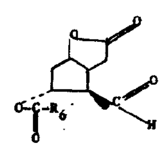

mit einem Phosphonester umsetzt.

Ihre Verwendung als Arzneimittel zur Beeinflussung hormonaler Reaktionen.


EP 0 000 710 A1


Croydon Printing Company Ltd.

BAYER AKTIENGESELLSCHAFT
Zentralbereich
Patente, Marken und Lizenzen

5090 Leverkusen, Bayerwerk
KS/AB
Ib (Pha)BEZEICHNUNG GEÄNDERT
siehe Titelseite

# Neue Prostaglandin-Analoga und Verfahren zu ihrer Herstellung

Die vorliegende Erfindung betrifft neue Prostaglandin-Analoga, ein Verfahren zu ihrer Herstellung sowie ihre Verwendung als Arzneimittel.
Prostaglandine sind bereits bekannt geworden (Deutsche Offenlegungsschrift 2 150 361).

Die physiologischen Wirkungen von Prostaglandinen sind jedoch sowohl in vitro als auch im Säuretierorganismus von kurzer Dauer, da sie schnell in pharmakologisch unwirksame Metabolite umgewandelt werden. Darüber hinaus ist es nachteilig, daß die Prostaglandine neben der gewünschten Wirkung gleichzeitig eine Reihe von unerwünschten physiologischen Nebenwirkungen besitzen, die ihre Verwendung als Arzneimittel stark einschränken.

Die Erfindung betrifft neue Prostaglandin-Analoga der allgemeinen Formel (I)

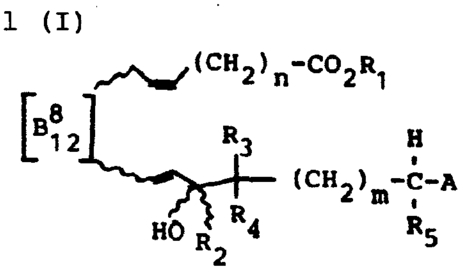

(I)

in welcher

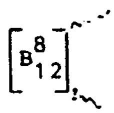 für die Teilstrukturen

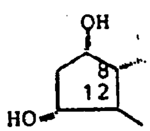
(Ia)

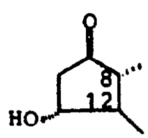
(Ib)

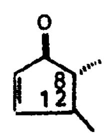 oder
(Ic)

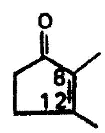
(Id)

steht.

$R_1$ für Wasserstoff oder einen gegebenenfalls substituierten Alkylrest steht,

$R_2$ für Wasserstoff oder einen Alkylrest steht,

$R_3$ und $R_4$ gleich oder verschieden sind und für Wasserstoff oder einen gegebenenfalls substituierten Alkylrest oder Aralkylrest stehen, wobei einer der Substituenten immer Wasserstoff ist, wenn der andere für Aralkyl steht,

$R_5$ für Wasserstoff oder einen gegebenenfalls substituierten Alkylrest oder Aralkylrest steht,

m für die Zahlen 0 bis 8 steht,

n für die Zahlen 2 bis 6 steht,

A für Cyano, Carboxy, Alkoxycarbonyl, N-disubstituiertes Carbamoyl steht und,

falls $R_1$ Wasserstoff bedeutet, auch deren physiologisch verträgliche Salze.

Die Schreibweise ~~~ bedeutet, dass die Substituenten $R_5$ und OH in α- oder β-Position stehen können;

die Schreibweise ..... bedeutet, dass die Substituenten α-ständig, die Schreibweise —— bedeutet, dass die Substituenten β-ständig angeordnet sind.

Die neuen Prostaglandin-Analoga können sowohl als Enantiomere, Enantiomerenpaare und Diastereomerenpaare vorliegen.

Es wurde gefunden, dass man Prostaglandin-Analoga der allgemeinen Formel (I) erhält, wenn man gemäß dem Reaktionsschema (I) einen Aldehyd der allgemeinen Formel (II)

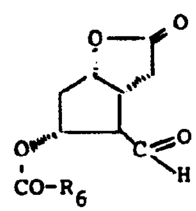

(II)

in welcher

$R_6$ für einen gegebenenfalls substituierten Alkyl- oder Arylrest steht, mit einem Phosphonester der allgemeinen Formel (III)

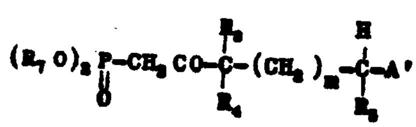

(III)

in welcher

$R_7$ für einen gegebenenfalls substituierten Alkylrest steht,

A' für Cyano, Alkoxycarbonyl oder N-disubstituiertes Carbamoyl steht und

$R_3$, $R_4$, $R_5$ und m die oben angegebene Bedeutung haben,

zu einem α, ß-ungesättigten Keton der Formel (IV)

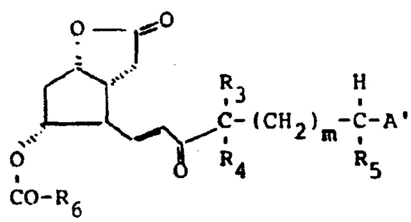

(IV)

in welcher
$R_3$, $R_4$, $R_5$, $R_6$, m und A' die oben genannte Bedeutung haben,

in Anwesenheit einer Base umsetzt und anschließend das α,β-ungesättigte Keton (IV) mit einem komplexen Metallborhydrid oder gegebenenfalls mit einer metallorganischen Alkylverbindung zu einem Allylalkohol der allgemeinen Formel (V)

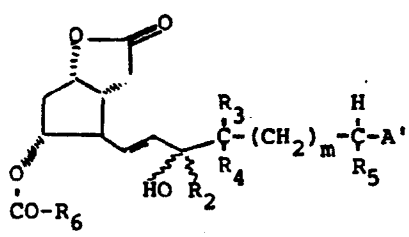

(V)

in welcher
$R_2$, $R_3$, $R_4$, $R_5$, $R_6$, m und A' die oben aufgeführte Bedeutung haben,

umsetzt, diesen durch Abspaltung der Gruppe $CO-R_6$ in ein Diol (VI) überführt, gegebenenfalls Schutzgruppen einführt und das so erhaltene Lacton der allgemeinen Formel (VII)

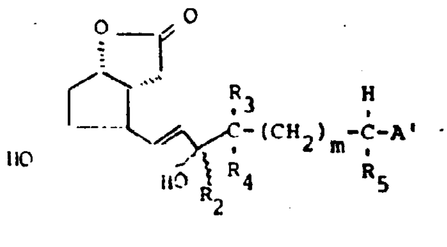

(VI)

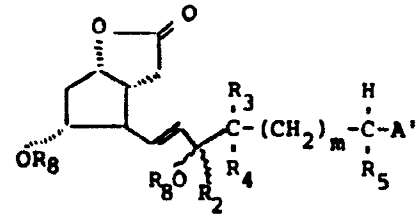

(VII)

in welcher

$R_8$ für einen Organosilyl - oder Ätherrest steht,

$R_2$, $R_3$, $R_4$, $R_5$, A' und m die obige Bedeutung haben,

zu einem Lactol der allgemeinen Formel (VIII)

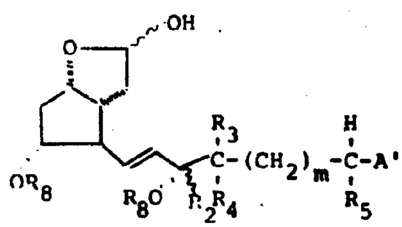

(VIII)

in welcher

$R_2$, $R_3$, $R_4$, $R_5$, $R_8$, A' und m die obige Bedeutung haben,

reduziert und das so erhaltene Lactol mit einem Phosphoniumsalz der allgemeinen Formel (IX)

$$\left[(R_9)_3{}^{\oplus}PCH_2(CH_2)_nCO_2H\right] X^{\ominus} \qquad (IX)$$

in welcher

**$R_9$ für einen Arylrest,**

**X für ein Halogen und**

**n für eine Zahl zwischen 2 und 6 stehen,**

**in Anwesenheit einer Base zu einer Säure der allgemeinen Formel (X)**

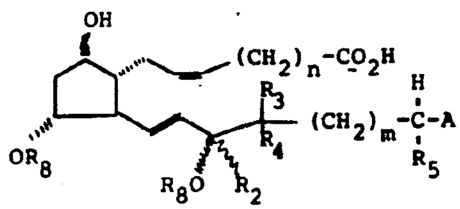 (X)

in welcher
$R_2$, $R_3$, $R_4$, $R_5$, $R_8$, A, m und n die obige Bedeutung haben,

umsetzt und anschließend für den Fall, daß in der Verbindung der allgemeinen Formel (I)

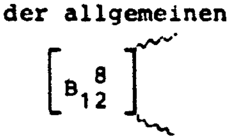 für 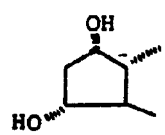 steht,

mit einer Säure gemäß dem Reaktionschema I die eingeführten Schutzgruppen, gegebenenfalls unter Veresterung, wieder abspaltet und so Verbindungen mit der allgemeinen Formel (Ia) erhält,

oder gemäß dem Reaktionsschema II für den Fall, daß in der Verbindung der allgemeinen Formel (I)

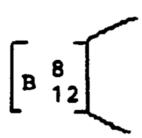 für 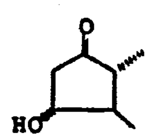 steht,

(X) zum Keton der allgemeinen Formel (XI)

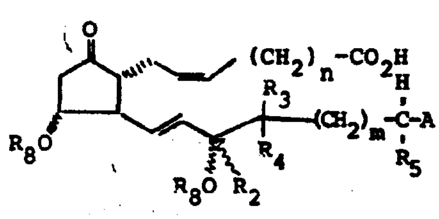

in welcher
$R_2$, $R_3$, $R_4$, $R_5$, $R_8$, A, m und n die obige Bedeutung haben,

oxidiert und anschließend die Schutzgruppen, gegebenenfalls unter Veresterung abspaltet und so Verbindungen der allgemeinen Formel (Ib) erhält,

oder gemäß dem Reaktionsschema II für den Fall, daß in der Verbindung der allgemeinen Formel (I)

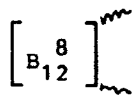 für 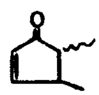 steht,

die Verbindung (Ib) oder (XI) mit einer Säure umsetzt und so die Verbindung der allgemeinen Formel (Ic) erhält,

oder gemäß dem Reaktionsschema II für den Fall, daß in der Verbindung der allgemeinen Formel (I)

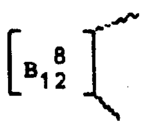 für 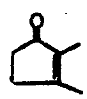 steht,

die Verbindung (Ib) mit einer Base umsetzt und so die Verbindung der allgemeinen Formel (Id) erhält.

<u>Reaktionsschema I</u>

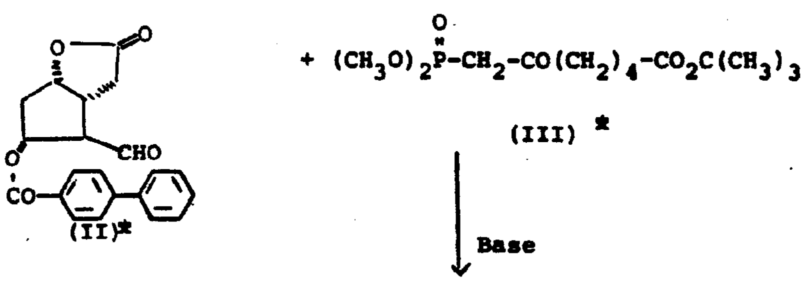

(II)*
(III)*

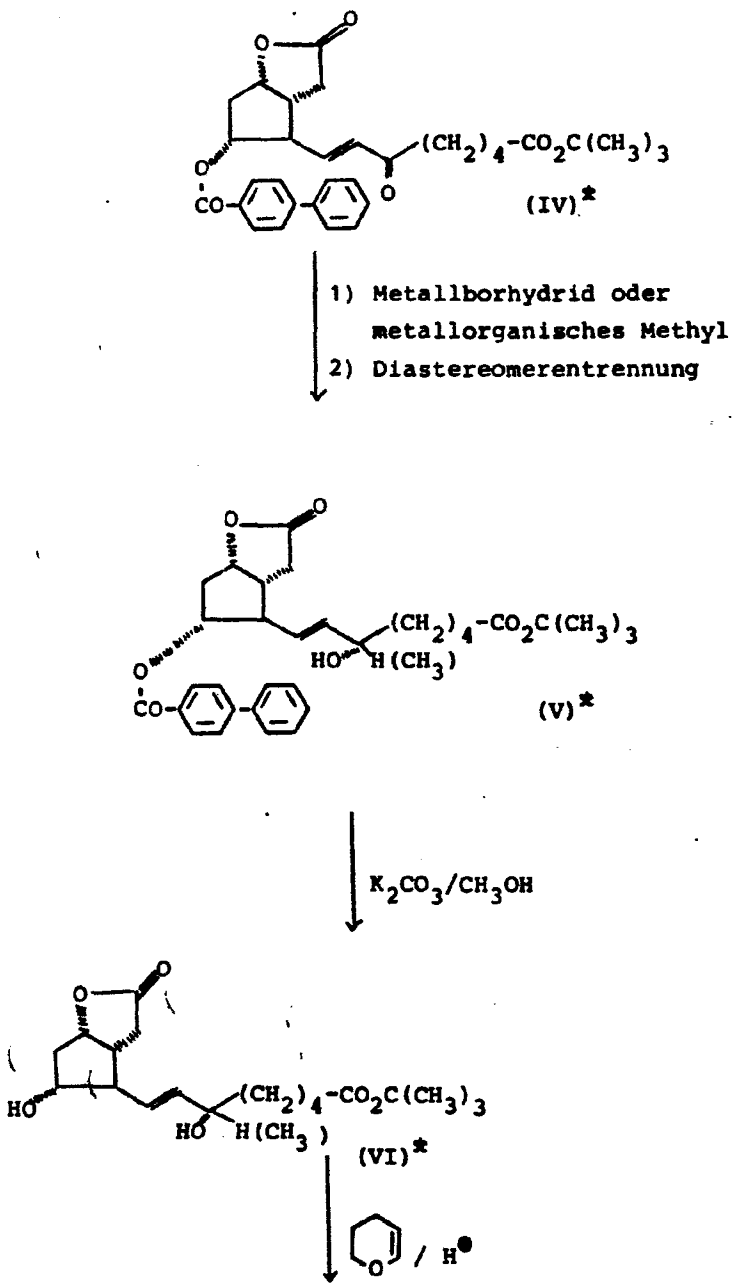
(IV)*
1) Metallborhydrid oder
metallorganisches Methyl
2) Diastereomerentrennung
(V)*
$K_2CO_3/CH_3OH$
(VI)*
/ $H^{\oplus}$

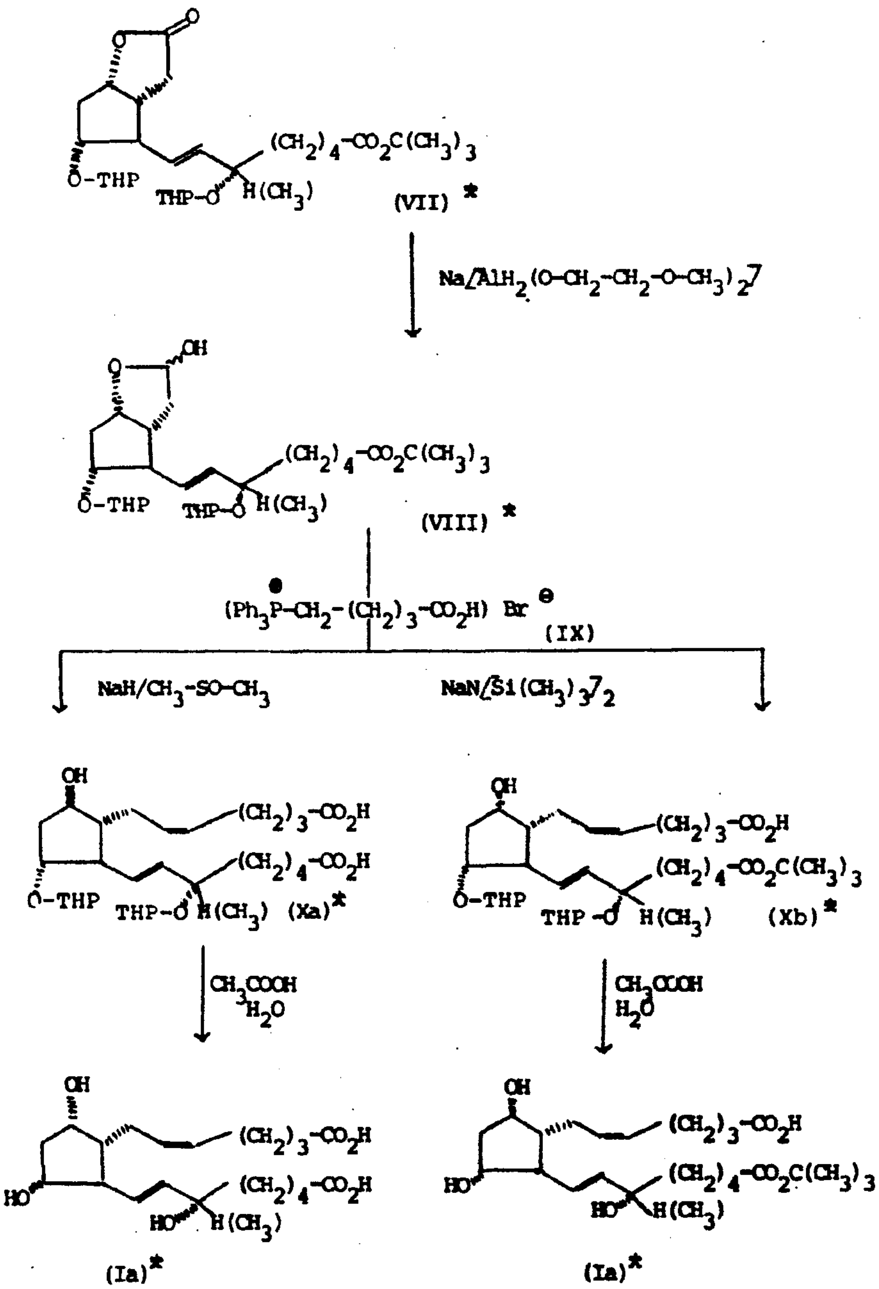


* bedeutet Auswahl aus der römisch bezifferten allgemeinen Formel

Reaktionsschema II

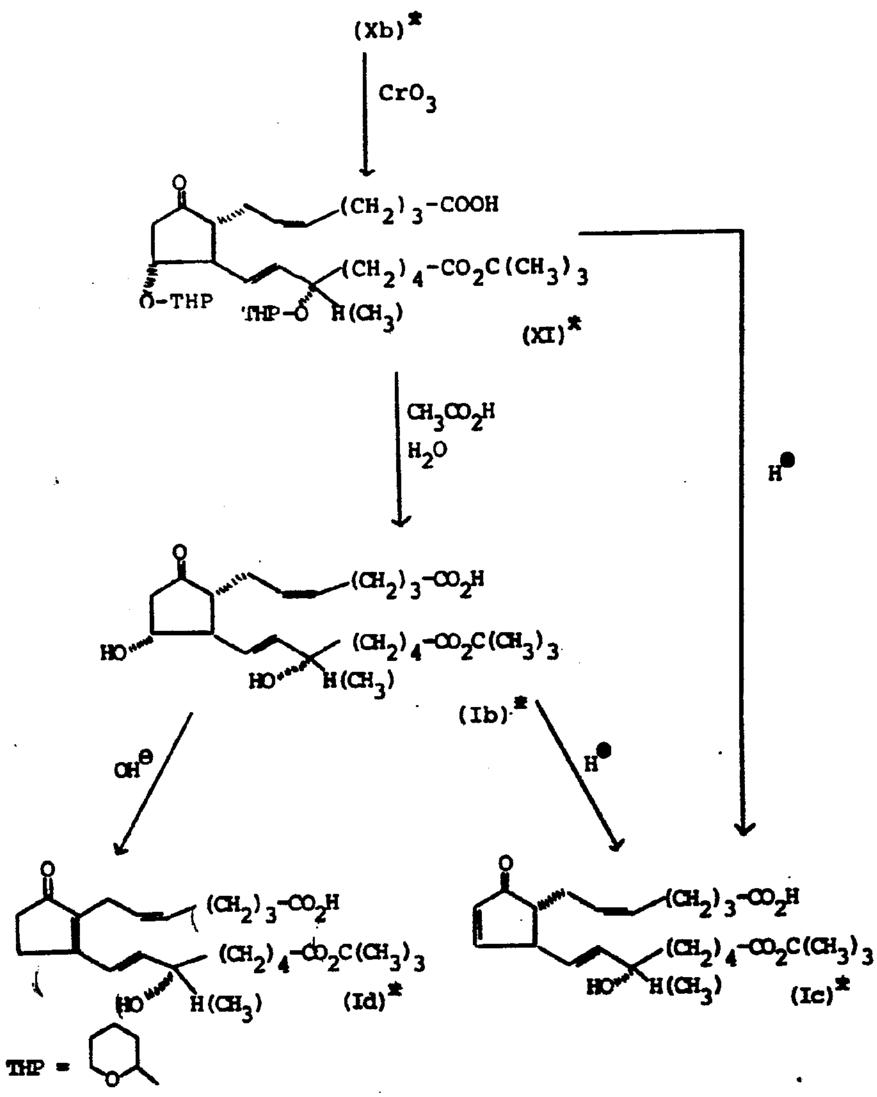


* bedeutet Auswahl aus der römisch bezifferten allgemeinen Formel

Entsprechend dem Reaktionsschema I wird in der ersten Stufe des Verfahrens ein Aldehyd der allgemeinen Formel (II) mit einem Phosphonester der allgemeinen Formel (III) in Gegenwart einer Base umgesetzt.

Die als Ausgangsstoffe verwendeten Aldehyde (II) sind bekannt oder können nach bekannten Verfahren hergestellt werden.
(J.Am.Chem.Soc. 1969, Bd.91, Seite 5675,
J.Am.Chem.Soc. 1970, Bd.92, Seite 397,
J.Am.Chem.Soc. 1971, Bd.93, Seite 1491).

In der Formel (II) steht $R_8$ vorzugsweise für Alkyl mit 1-4 Kohlenstoffatomen, insbesondere für Methyl und vorzugsweise für Aryl, insbesondere Phenyl und p-Diphenyl. Beispielsweise seien folgende Verbindungen genannt:
2-Oxa-3-oxo-6-formyl-7-(acetyloxy-bicyclo[3,3,0]octan,
2-Oxa-3-oxo-6-formyl-7-(p-phenylbenzoyloxy)-bicyclo[3,3,0]octan,
2-Oxa-3-oxo-6-formyl-7-(benzoyloxy)-bicyclo[3,3,0]octan.

Die weiterhin als Ausgangsstoffe verwendeten Phosphonester der allgemeinen Formel (III) sind bislang noch nicht bekannt, können aber nach grundsätzlich bekannten Verfahren hergestellt werden.
(J.Am. Chem.Soc. 1966, Bd. 88, Seite 1966).

In der Formel (III) stehen vorzugsweise
$R_3$, $R_4$ und $R_5$ für Wasserstoff, Benzyl und Alkyl mit 1 bis 4 Kohlenstoffatomen, insbesondere für Wasserstoff und Alkyl mit 1 bis 2 Kohlenstoffatomen,
$R_7$ für einen Alkylrest mit 1 bis 4 Kohlenstoffatomen, insbesondere für einen Alkylrest mit 1 bis 2 Kohlenstoffatomen,
m vorzugsweise für eine Zahl von 0 bis 6.

In der Gruppierung A' kann die Alkoxycarbonylgruppe einen $C_1$-$C_8$ Alkoxyrest besitzen, der auch verzweigt sein kann.

Vorzugsweise seien genannt:
Verzweigte $C_3$ bis $C_8$-Alkoxyreste, insbesondere der tert.-Butyloxyrest und der iso-Propyloxyrest.

Weiterhin kann in der Gruppierung A' das N-disubstituierte Carbamoyl am Stickstoff durch gegebenenfalls verzweigte Alkylgruppen von 1 bis 8 Kohlenstoffatomen, vorzugsweise von 1-5 Kohlenstoffatomen, zum Beispiel Methyl, substituiert sein. Diese am N befindlichen Substituenten können auch, gegebenenfalls über weitere Heteroatome wie Sauerstoff oder Schwefel mit dem N-Atom einen heterocyclischen Ring bilden. Vorzugsweise seien genannt:
Piperidin, Pyrrolidin, Morpholin, Cyclohexylimin.

Weiter kann A' für eine Cyanogruppe stehen.

Beispielsweise seien folgende Verbindungen, die der allgemeinen Formel (III) entsprechen, genannt:

10-tert-Butoxycarbonyl-2-oxo-decanphosphonsäuredimethylester,
10-tert-Butoxycarbonyl-2-oxo-3-methyl-decanphosphonsäuredimethylester,
10-tert-Butoxycarbonyl-2-oxo-3,3-dimethyl-decanphosphonsäuredimethylester,
6-tert-Butoxycarbonyl-2-oxo-hexanphosphonsäuredimethylester,
6-tert-Butoxycarbonyl-2-oxo-3-methyl-hexanphosphonsäuredimethylester,
6-tert-Butoxycarbonyl-2-oxo-3,3-dimethylhexanphosphonsäuredimethylester,

6-iso-Propyloxycarbonyl-2-oxo-hexanphosphonsäuredimethylester,
6-iso-Propyloxycarbonyl-3,3-dimethyl-2-oxo-hexanphosphonsäuredimethylester,

8-Cyano-2-oxo-octanphosphonsäuredimethylester,
7-Cyano-2-oxo-heptanphosphonsäuredimethylester,
7-Cyano-3-methyl-2-oxo-heptanphosphonsäuredimethylester,
7-Cyano-3,3-dimethyl-2-oxo-heptanphosphonsäuredimethylester,
6-Cyano-2-oxo-hexanphosphonsäuredimethylester,
6-Cyano-3-methyl-2-oxo-hexanphosphonsäuredimethylester,
6-Cyano-3,3-dimethyl-2-oxo-hexanphosphonsäuredimethylester,
5-Cyano-2-oxo-pentanphosphonsäuredimethylester,
5-Cyano-3-äthyl-2-oxo-pentanphosphonsäuredimethylester,
5-Cyano-3,3-dimethyl-2-oxo-pentanphosphonsäuredimethylester,
5-Cyano-2-oxo-hexanphosphonsäuredimethylester,
4-Cyano-2-oxo-butanphosphonsäuredimethylester,

8-Piperidinocarbonyl-2-oxo-octanphosphonsäuredimethylester,
8-Piperidinocarbonyl-3-methyl-2-oxo-octanphosphonsäuredimethylester,
8-Piperidinocarbonyl-3,3-dimethyl-2-oxo-octanphosphonsäuredimethylester,
4-Piperidinocarbonyl-2-oxo-butanphosphonsäuredimethylester,
4-Piperidinocarbonyl-3,3-dimethyl-2-oxo-butanphosphonsäuredimethylester,

8-Dimethylaminocarbonyl-2-oxo-octanphosphonsäuredimethylester,
4-Dimethylaminocarbonyl-2-oxo-butanphosphonsäuredimethylester,
4-Dimethylaminocarbonyl-3,3-dimethyl-2-oxo-butanphosphonsäuredimethylester,
4-(4-Methyl-3-piperideinocarbonyl)-2-oxo-butanphosphonsäuredimethylester,
4-Pyrrolidinocarbonyl-2-oxo-butanphosphonsäuredimethylester,
4-Morpholinocarbonyl-2-oxo-butanphosphonsäuredimethylester.

Als gegebenenfalls verwendete Base eignen sich metallorganische Verbindungen wie Butyllithium oder tert-Butyllithium, vorzugsweise n-Butyllithium oder Metallhydride wie Lithiumhydrid, Natriumhydrid oder Calciumhydrid, vorzugsweise Natriumhydrid, Alkoxide wie Natriummethylat, Kaliummethylat, Natrium-tert-butylat, Kalium-tert-butylat, vorzugsweise Natrium- oder Kalium-tert-butylat oder Metallamide wie Natriumamid, Kaliumamid, vorzugsweise Natriumamid oder auch Diazabicycloalkene wie 1,5-Diazabicyclo[4,3,0]non-5-en, 1,8-Diazabicyclo[5,4,0]undec-7-en, 1,5-Diazabicyclo[4,4,0]dec-5-en, 1,8-Diazabicyclo[5,3,0]dec-7-en, vorzugsweise 1,5-Diazabicyclo[4,3,0]non-5-en (DBN) und 1,8-Diazabicyclo[5,4,0]undec-7-en (DBU).

Das erfindungsgemäße Verfahren wird in der ersten Stufe in Gegenwart eines für die Reaktionspartner inerten Lösungsmittels durchgeführt. Die Wahl des Lösungsmittels hängt von der gegebenenfalls verwendeten Base ab. Geeignete Lösungsmittel sind beispielsweise Aether wie Tetrahydrofuran, Diäthyläther oder Dimethoxyäthan, Nitrile wie Acetonitril oder Propionitril, Amide wie Dimethylformamid, Dimethylacetamid oder heterocyclische Verbindungen wie N-Methylpyrrolidon oder Phosphorsäure-Derivate wie Hexamethylphosphorsäuretriamid.

Das erfindungsgemäße Verfahren wird in der ersten Stufe im Temperaturbereich -20°C und +50°C, vorzugsweise +15°C und 30°C durchgeführt.

Im allgemeinen setzt man 1 Mol der Verbindung (II) mit 1,0 bis 1,5 Mol, vorzugsweise 1,05 bis 1,3 Mol des Phosphonesters (III), der vorher mit 1,0 bis 1,2 Mol, vorzugsweise 1,05 bis 1,15 Mol Base umgesetzt wurde, um.

Die Reaktionszeit ist von der Temperatur abhängig und liegt im allgemeinen zwischen einer halben Stunde und 3 Stunden. Im allgemeinen wird das beschriebene Verfahren unter Normaldruck durchgeführt.

Entsprechend dem Reaktionsschema 1 : in der zweiten Stufe des Verfahrens das erhaltene α,β-ungesättigte Keton der allgemeinen Formel (IV) mit einem komplexen Metallborhydrid umgesetzt. Als komplexe Metallborhydride eignen sich Metallborhydride wie Natriumborhydrid, vorzugsweise Zinkborhydrid oder auch Metallorganylborhydride wie Lithium-tris-isoamylborhydrid, Lithium-perhydro-9b-boraphenalylhydrid, Lithium-9-tert-butyl-9-borabicyclo[3,3,1] nonylhydrid, Lithium-diisopinocamphenyl-tert-butylborhydrid, Lithium-2-thexyl-4,8-dimethyl-2-borbicyclo[3,3,1]nonylhydrid, Kalium-tris-sek-butylborhydrid (Kaliumselectrid)

Die Reaktion wird in Gegenwart eines für die Reaktionspartner inerten Lösungsmittels durchgeführt. Die Wahl des Lösungsmittels ist abhängig vom Reduktionsmittel. Als Lösungsmittel eignen sich beispielsweise Aether wie Diäthyläther, Tetrahydrofuran oder Dimethoxy-äthan oder Kohlenwasserstoffe wie Toluol oder auch Gemische der infrage kommenden Lösungsmittel.

Die Temperatur liegt zwischen -130°C und +20°C, vorzugsweise zwischen -105°C und 0°C.

Im allgemeinen setzt man 1 Mol der Verbindung (IV) mit mindestens 1 Hydridäquivalent des Reduktionsmittels um. Ein Überschuß schadet nicht.

Beim Einsatz von Zinkborhydrid ist es nicht erforderlich, das Reduktionsmittel als solches einzusetzen. Es genügt, Zinkborhydrid aus Natriumborhydrid und Zinkchlorid herzustellen und diese das Reduktionsmittel enthaltene Lösung als solche einzusetzen.

Die Reaktionszeit ist von der Temperatur abhängig und liegt im allgemeinen zwischen 1 und 20 Stunden.

Reduziert man das α,β-ungesättigte Keton der allgemeinen Formel (IV) mit Metallborhydriden wie Natriumborhydrid oder vorzugsweise mit Zinkborhydrid so erhält man Diastereomerengemische, die gegebenenfalls durch Chromatographie aufgetrennt werden können.

Reduziert man das Keton (IV) mit Metallborhydriden wie Lithium-tris-isoamylborhydrid, Lithiumperhydro-9b-boraphenalyl-hydrid, Lithium-9-tert-butyl-9-borabicyclo[3,3,1]nonylhydrid Lithium-diisopinocamphenyl-tert-butylborhydrid, Lithium-2-thexyl-4,8-dimethyl-2-borbicyclo[3,3,1]nonylhydrid oder Kaliumselectrid, so kann gegebenenfalls auf die Diastereomerentrennung, beispielsweise durch Chromatographie verzichtet werden, da vorwiegend bis nahezu ausschließlich nur ein Diastereomeres entsteht.

In einer Verfahrensvariante wird das α,β-ungesättigte Keton der allgemeinen Formel (IV) mit einer metallorganischen Alkylverbindung umgesetzt.

Als metallorganische Alkylverbindungen eignen sich Grignard-Verbindungen mit einem Alkylrest von 1 bis 6 Kohlenstoffatomen, vorzugsweise mit 1 bis 2 Kohlenstoffatomen oder Lithiumalkylverbindungen mit einem Alkylrest von 1 bis 6 Kohlenstoffatomen, vorzugsweise mit 1 bis 2 Kohlenstoffatomen.

Insbesondere seien genannt:

Methylmagnesiumchlorid, Methylmagnesiumbromid, Methylmagnesiumjodid, Methyllithium, Aethylmagnesiumbromid.

Die Reaktion wird in Gegenwart eines für die Reaktionspartner inerten Lösungsmittels durchgeführt. Als Lösungsmittel eignen sich beispielsweise Aether wie Diäthyläther, Tetrahydrofuran oder Dimethoxyäthan oder Gemische der infrage kommenden Lösungsmittel.

Die Temperaturen liegen zwischen -90°C und 0°C, vorzugsweise zwischen -78°C und -10°C.

Im allgemeinen setzt man 1 Mol der Verbindung (IV) mit 1,0 bis 3 Mol der metallorganischen Alkylverbindung, vorzugsweise mit 1,0 bis 2 Mol um. Die Reaktionsdauer ist von der Temperatur abhängig und liegt zwischen 1 bis 2 Stunden.

Das bei den Alkylierungen α,β-ungesättigter Ketone der allgemeinen Formel (IV) mit metallorganischen Alkylverbindungen wie Methylmagnesiumjodid anfallende Diastereomerengemisch läßt sich gegebenenfalls durch Chromatographie auftrennen.

Entsprechend dem Reaktionsschema I wird in einer dritten Stufe des Verfahrens der Allylalkohol der allgemeinen Formel (V) mit Alkalihydroxiden oder Alkalicarbonaten in Wasser oder Alkoholen, vorzugsweise mit Alkalicarbonaten in Alkoholen umgesetzt. Als Alkalicarbonate eignen sich beispielsweise Kalium- oder Natriumcarbonat insbesondere Kaliumcarbonat. Als Alkohole eignen sich vorzugsweise solche mit einem geradkettigen Alkylrest mit 1-4 Kohlenstoffatomen insbesondere Methanol und Äthanol.

Das erfindungsgemäße Verfahren wird in der dritten Stufe im Temperaturbereich von 0°C und 50°C, vorzugsweise von 20°C und 30°C durchgeführt. Die Reaktionszeit ist von der Temperatur abhängig und liegt im allgemeinen zwischen einer Stunde und drei Stunden.

Im allgemeinen setzt man 1 Mol des Allylalkohols der allgemeinen Formel (V) mit 1,0 bis 1,2 Mol Alkalicarbonat, vorzugsweise mit 1,0 bis 1,1 Mol um.

In einer vierten Stufe des erfindungsgemäßen Verfahrens führt man gegebenenfalls Schutzgruppen entsprechend dem Reaktionsschema I in das Diol der allgemeinen Formel (VI) ein. Zur Einführung von Schutzgruppen eignen sich acyclische wie cyclische Enoläther, beispielsweise Dihydropyran und Aethylvinyläther, insbesondere Dihydropyran

Als Verdünnungsmittel kommen alle inerten organischen Lösungsmittel in Frage. Hierzu gehören vorzugsweise chlorierte Kohlenwasserstoffe wie Methylenchlorid, Chloroform und Aether wie Diäthyläther, Tetrahydrofuran oder Dimethoxyäthan.

Als Katalysatoren kommen alle üblichen Säuren in Frage. Hierzu gehören vorzugsweise Salzsäure, Schwefelsäure, Oxalsäure, p-Toluolsulfonsäure, insbesondere p-Toluolsulfonsäure.

Die Reaktionstemperaturen können über einen größeren Bereich variiert werden. Im allgemeinen arbeitet man zwischen etwa 0°C und 50°C, vorzugsweise zwischen 20°C und 30°C.

Im allgemeinen setzt man 1 Mol des Diols (VI) mit mindestens 2 Mol Enoläther um. Ein Ueberschuß schadet nicht.

In einer Verfahrensvariante wird das Diol der allgemeinen Formel (VI) mit Trialkylsilylhalogeniden in Anwesenheit eines Säurebinders umgesetzt.

Als Trialkylsilylhalogenide eignen sich Trimethylchlorsilan und tert-Butyl-dimethyl-chlorsilan, vorzugsweise tert-Butyl-dimethyl-chlorsilan.

Als Säurebinder können alle üblichen Säurebindungsmittel verwendet werden. Hierzu gehören vorzugsweise aliphatische tertiäre Amine und aromatische N-Heterocyclen. Als besonders geeignet sei Imidazol genannt.

Als Lösungsmittel eignen sich beispielsweise aliphatische Amide wie Dimethylformamid, Dimethylacetamid, Hexamethylphosphorsäuretriamid, insbesondere Dimethylformamid.

Die Temperaturen liegen zwischen 10°C und 80°C, vorzugsweise zwischen 20°C und 50°C. Die Reaktionsdauer ist von der Temperatur abhängig und liegt zwischen 6 und 24 Stunden.

Bei der Durchführung des erfindungsgemäßen Verfahrens setzt

man 1 Mol des Diols (VI) mit 2,2 [illegible] 2,4 bis 3,0 Mol tert. Bu[illegible] heit von 4,4 bis 8,0 Mol, vorzugswe[illegible] Mol Imid-azol um.

In einer vierten Stufe des erfindungsgemäßen Verfahrens redu-ziert man gemäß Reaktionsschema [illegible] der allgeme[illegible] Formel (VII) mit Alkali-alkoxy-hyd[illegible] aluminaten, beispiels-weise Natrium-bis-(2-methoxy-äthox[illegible] aluminat oder Natrium-äthoxy-bis-(2-methoxy-äthox[illegible] minat.

Ein besonders geeignetes Reduktionsm[illegible] ist das Natrium-bis-(2-methoxy-äthoxy)-dihydridoaluminat[illegible] Verdünnungsmittel eignet sich Toluol. Die Reaktionstemperaturen liegen zwischen -78°C und -40°C, vorzugsweise zwische[illegible] 78°C und -70°C. Die Reaktionszeit hängt von der Reaktionstemperatur ab und liegt zwischen vier und zehn Stunden. Im [illegible] gemeinen setzt man 1 Mol des Lactons (VII) mit mindestens [illegible] däquivalent des Reduktionsmittels um. Ein Ueberschuß schadet nicht.

In einer fünften Stufe des erfindungsgemäßen Verfahrens wird entsprechend dem Reaktionsschema [illegible] ctol der allgemei-nen Formel (VIII) mit einem Phosph[illegible] umsalz der allgemeinen Formel (IX) in Gegenwart einer Base umgesetzt

Die als Ausgangsstoffe verwendeten Phosphoniumsalze sind be-kannt (J.Am. Chem.Soc. 1969, Bd [illegible] 5675

In der Formel (IX) steht n vorzugs[illegible] 2 bis 4 und x für Cl, Br, J, insbesondere [illegible]

Beispielsweise seien folgende [illegible] genannt

3-Carboxy-propyl-triphenylphosp[illegible]

4-Carboxy-butyl-triphenylphosp[illegible]

5-Carboxy-pentyl-triphenylphosp[illegible]

4-Carboxy-butyl-triphenylphosp[illegible]



BAD ORIGINAL

Als gegebenenfalls verwendete Base eignen sich Alkalihydride wie Natriumhydrid, Kaliumhydrid, vorzugsweise Natriumhydrid, Alkali-bis-(trialkylsilyl)amide wie Natrium-bis-(trialkylsilyl)-amide mit einem Alkyl von 1-4 Kohlenstoffatomen, insbesondere Natrium-bis-(trimethylsilyl)-amid.

Die Wahl des Lösungsmittels hängt von der gegebenenfalls verwendeten Base ab. Verwendet man Alkali-bis-(trialkylsilyl)-amide als Base, so führt man die Reaktion in einem inerten Lösungsmittel wie Aether, beispielsweise Diäthyläther, Tetrahydrofuran, Dialkoxyäthan, insbesondere Dimethoxyäthan durch. Die Reaktionstemperatur liegt in einem Temperaturbereich von 0°C und 40°C, vorzugsweise von 5°C und 30°C.

Die Reaktionszeit ist von dem Temperaturbereich abhängig und liegt im allgemeinen zwischen einer Stunde und 4 Stunden.

Verwendet man Alkalihydrid als Base, so führt man die Reaktion in Dimethylsulfoxid als Lösungsmittel durch. Steht in der allgemeinen Formel (VIII) A' für tert-Butoxycarbonyl, so tritt gegebenenfalls Hydrolyse zur Carboxygruppe ein.

Die Reaktionstemperatur liegt zunächst in einem Temperaturbereich zwischen 30°C und 90°C, vorzugsweise von 60°C und 70°C (Herstellung des Natriumsalzes des Dimethylsulfoxids) und anschließend in einem Temperaturbereich zwischen 10°C und 40°C, vorzugsweise 15°C und 25°C. Die Reaktionszeit ist von der Temperatur abhängig und liegt im allgemeinen zwischen einer Stunde und zwei Stunden.

Im allgemeinen setzt man 1 Mol des Lactols (VIII) mit 1,0 bis 3,0 Mol, vorzugsweise 1,3 bis 2,8 Mol eines Phosphoniumsalzes der allgemeinen Formel (IX) um, das zunächst mit 2,0 bis 6,0 Mol, vorzugsweise 2,6 bis 5,6 Mol Base umgesetzt wurde.

In einer sechsten Stufe des erfindungsgemäßen Verfahrens wird entsprechend dem Reaktionsschema I die Verbindung der allgemeinen Formel (X) mit einer Säure behandelt.

Als Säuren eignen sich niedere aliphatische Mono- und Dicarbonsäuren oder auch niedere aliphatische Hydroxy-tri-carbonsäuren. Beispielsweise seien genannt:

Essigsäure, Propionsäure, Oxalsäure, Zitronensäure. Vorzugsweise verwendet man Essigsäure. Die Reaktion wird in einem organischen Lösungsmittel in Gegenwart von Wasser durchgeführt. Es eignen sich solche Lösungsmittel, die mit Wasser mischbar und gegenüber den eingesetzten Säuren inert sind. Es seien beispielsweise genannt:

Tetrahydrofuran oder Dioxan.
Als Lösungsmittel eignen sich auch gegebenenfalls die verwendeten Säuren, beispielsweise Essigsäure. Vorzugsweise werden Gemische aus Wasser, niederer aliphatischer Säure und organischem Lösungsmittel verwendet. Als Lösungsmittel eignen sich weiter niedere Alkohole wie Methanol, Aethanol, Propanol, vorzugsweise Methanol.

Die Zusammensetzung des Gemisches ist nicht kritisch, die Verbindung (X) soll jedoch darin löslich sein. Zweckmäßig verwendet man einen größeren Ueberschuß an Säure. Die Temperatur liegt zwischen +20°C und +60°C, vorzugsweise bei +30°C bis +55°C. Die Reaktionsdauer ist von der Temperatur abhängig und liegt zwischen 2 und 10 Stunden.

Als neue Wirkstoffe gemäß Reaktionsschema I seien beispielhaft genannt:

(5Z, 9α, 11α, 13E, 15α)-9,11,15-Trihydroxy-23-tert-butoxy=carbonyl-21,22,23-tri-homo-prosta-5,13-dien-1-säure,
(5Z,9α, 11α, 13E, 15α)-9,11,15-Trihydroxy-16-methyl-23-tert-butoxy-carbonyl-21,22,23-tri-homo-prosta-5,13-dien-1-säure,
(5Z, 9α, 11α, 13E, 15α)-9,11,15-Trihydroxy-16,16-dimethyl-23-tert-butoxycarbonyl-21,22,23-tri-homo-prosta-5,13-dien-1-säure,
(5Z, 9α, 11α, 13E, 15α)-9-11,15-Trihydroxy-19-tert-butoxycar=bonyl-20-nor-prosta-5,13-dien-1-säure,
(5Z, 9α, 11α, 13E, 15α)-9,11,15-Trihydroxy-15-β-methyl-19-tert-butoxycarbonyl-20-nor-prosta-5,13-dien-1-säure,
(5Z, 9α, 11α, 13E, 15α)-9,11,15-Trihydroxy-16-methyl-19-tert-butoxycarbonyl-20-nor-prosta-5,13-dien-1-säure,
(5Z, 9α, 11α, 13E, 15α)-9,11,15-Trihydroxy-16,16-dimethyl-19-tert-butoxycarbonyl-20-nor-prosta-5,13-dien-1-säure,
(5Z, 9α, 11α, 13E, 15α)-9,11,15-Trihydroxy-19-iso-propoxy=carbonyl-20-nor-prosta-5,13-dien-1-säure,
(5Z, 9α, 11α, 13E, 15α)-9,11,15-Trihydroxy-16,16-dimethyl-19-iso-propoxycarbonyl-20-nor-prosta-5,13-dien-1-säure,
(5Z, 9α, 11α, 13E, 15α)-9,11,15-Trihydroxy-19-carboxy-20-nor-prosta-5,13-dien-1-säure,
(5Z, 9α, 11α, 13E, 15α)-9,11,15-Trihydroxy-16,16-dimethyl-19-carboxy-20-nor-prosta-5,13-dien-1-säure,
(5Z, 9α, 11α, 13E, 15α)-9,11,15-Trihydroxy-21-cyano-21-homo-prosta-5,13-dien-1-säure,
(5Z, 9α, 11α, 13E, 15α)-9,11,15-Trihydroxy-15β-methyl-21-cyano-21-homo-prosta-5,13-dien-1-säure,
(5Z, 9α, 11α, 13E, 15α)-9,11,15-Trihydroxy-20-cyano-prosta-5,13-dien-1-säure,
(5Z, 9α, 11α, 13E, 15α)-9,11,15-Trihydroxy-16-methyl-20-cyano-prosta-5,13-dien-1-säure,
(5Z,9α,11α, 13E, 15α)-9,11,15-Trihydroxy-16,16-dimethyl-20-cyano-prosta-5,13-dien-1-säure,
(5Z, 9α, 11α, 13E, 15α)-9,11,15-Trihydroxy-19-cyano-20-nor-prosta-5,13-dien-1-säure,
(5Z, 9α, 11α, 13E, 15α)-9,11,15-Trihydroxy-16-methyl-19-cyano-20-nor-prosta-5,13-dien-1-säure,

(5Z, 9α, 11α, 13E, 15α)-9, 11,15-Trihydroxy-16,16-dimethyl-19-cyano-20-nor-prosta-5,13-dien-1-säure,

(5Z, 9α, 11α, 13E, 15α)-9,11,15-Trihydroxy-18-cyano-19,20-di-nor-prosta-5,13-dien-1-säure,

(5Z, 9α, 11α, 13E, 15α)-9,11,15-Trihydroxy-16-äthyl-18-cyano-19,20-di-nor-prosta-5,13-dien-1-säure,

(5Z, 9α, 11α, 13E, 15α)-9,11,15-Trihydroxy-16,16-dimethyl-18-cyano-19,20-di-nor-prosta-5,13-dien-1-säure,

(5Z, 9α, 11α, 13E, 15α)-9,11,15-Trihydroxy-18-cyano-20-nor-prosta-5,13-dien-1-säure,

(5Z, 9α, 11α, 13E, 15α)-9,11,15-Trihydroxy-17-cyano-18,19,20-tri-nor-prosta-5,13-dien-1-säure,

(5Z, 9α, 11α, 13E, 15α)-9,11,15-Trihydroxy-21-piperidinocarbonyl-21-homo-prosta-5,13-dien-1-säure,

(5Z, 9α, 11α, 13E, 15α)-9,11,15-Trihydroxy-15β-methyl-21-piperidinocarbonyl-21-homo-prosta-5,13-dien-1-säure,

(5Z, 9α, 11α, 13E, 15α)-9,11,15-Trihydroxy-16-methyl-21-piperidinocarbonyl-21-homo-prosta-5,13-dien-1-säure,

(5Z, 9α, 11α, 13E, 15α)-9,11,15-Trihydroxy-16,16-dimethyl-21-piperidinocarbonyl-21-homo-prosta-5,13-dien-1-säure,

(5Z, 9α, 11α, 13E, 15α)-9,11-15-Trihydroxy-17-piperidinocarbonyl-18,19,20-tri-nor-prosta-5,13-dien-1-säure,

(5Z, 9α, 11α, 13E, 15α)-9,11,15-Trihydroxy-16,16-dimethyl-17-piperidinocarbonyl-18,19-20-tri-nor-prosta-5,13-dien-1-säure,

(5Z, 9α, 11α, 13E, 15α)-9,11,15-Trihydroxy-21-dimethyl-aminocarbonyl-21-homo-prosta-5,13-dien-1-säure,

(5Z, 9α, 11α, 13E, 15α)-9,11,15-Trihydroxy-17-dimethylaminocarbonyl-18,19,20-tri-nor-prosta-5,13-dien-1-säure,

(5Z, 9α, 11α , 13E, 15α)-9,11,15-Trihydroxy-16,16-dimethyl-17-dimethylaminocarbonyl-18,19,20-tri-nor-prosta-5,13-dien-1-säure,

(5Z. 9α. 11α. 13E. 15α)-9,11,15-Trihydroxy-17-(4-methyl-$\Delta^3$-piperideinocarbonyl)-18,19,20-tri-nor-prosta-5,13-dien-1-säure,

(5Z, 9α, 11α, 13E, 15α)-9,11,15-Trihydroxy-17-pyrrolidinocarbonyl-18,19,20-tri-nor-prosta-5,13-dien-1-säure,

(5Z, 9α, 11α, 13E, 15α)-9,11,15-Trihydroxy-17-morpholinocarbonyl-18,19,20-tri-nor-prosta-5,13-dien-1-säure,

ihre 15β-Diastereomeren beziehungsweise nach bekannten Verfahren hergestellen entsprechenden Ester.

Entsprechend dem Reaktionsschema II wird in einer weiteren Stufe des erfindungsgemäßen Verfahrens eine Verbindung der allgemeinen Formel (X) mit sechswertigem Chrom oxidiert.

Ein geeignetes Oxidationsmittel ist das Jones-Reagens, das heißt angesäuerte Chromsäure (Journal of the Chemical Society 1947, Seite 39). Als Verdünnungsmittel eignet sich Aceton. Zweckmäßig setzt man 1 Mol der Verbindung mit der stöchiometrisch berechneten Menge beziehungsweise mit einem bis zu [illegible] Ueberschuß an Oxidationsmittel um. Die Reaktionstemperaturen liegen zwischen -70°C und + 20°C, zweckmäßig bei -20°C bis 0°C.

Ebenso geeignet für die obige Reaktion ist das Collins-Reagens, das heißt Chromtrioxid in Pyridin (Tetrahedron Letters 1968, Seite 3363). Als Verdünnungsmittel eignet sich Methylenchlorid. Zweckmäßig setzt man 1 Mol der Verbindung mit dem 5- bis 10-fachen Ueberschuß, vorzugsweise mit dem 6- bis 8-fachen Ueberschuß der
Oxidation einer sekundären Hydroxylgruppe an Oxidationsmittel um. Die Reaktionstemperatur liegt zwischen -20°C bis +30°C, vorzugsweise zwischen -10°C bis +10°C.

In einer weiteren Stufe des erfindungsgemäßen Verfahrens wird entsprechend Reaktionsschema II die Verbindung der allgemeinen Formel (XI) mit einer Säure behandelt.

Als Säuren eignen sich niedere aliphatische Mono- und Dicarbonsäuren oder auch niedere aliphatische Hydroxy-tricarbonsäuren; beispielsweise seien genannt:

Essigsaure, Propionsaure, Oxalsäure, Zitronensäure. Vorzugsweise verwendet man Essigsäure. Die Reaktion wird in einem organischen Lösungsmittel in Gegenwart von Wasser durchgeführt. Es eignen sich solche Lösungsmittel, die mit Wasser mischbar und gegenuber den eingesetzten Säuren inert sind. Es seien beispielsweise genannt:
Tetrahydrofuran oder Dioxan. Als Lösungsmittel eignen sich auch gegebenenfalls die verwendeten Säuren, beispielsweise Essigsäure. Vorzugsweise werden Gemische aus Wasser, niederer aliphatischer Säure und organischem Lösungsmittel verwendet.

Als Lösungsmittel eignen sich weiter niedere Alkohole wie Methanol, Aethanol, Propanol, vorzugsweise Methanol.

Die Zusammensetzung des Gemisches ist nicht kritisch, die Verbindung (XI) soll jedoch darin löslich sein. Zweckmäßig verwendet man einen größeren Ueberschuß an Säure. Die Temperatur liegt zwischen +20°C bis +60°C, vorzugsweise bei +30°C bis +55°C. Die Reaktionsdauer ist von der Temperatur abhangig und liegt zwischen 2 bis 10 Stunden.

Als neue erhaltene Wirkstoffe gemäß Reaktionsschema II seien beispielhaft genannt:

(5Z,11α,13E, 15α)-9-Oxo-11,15-dihydroxy-23-tert-butoxycarbonyl-21,22,23-tri-homo-prosta-5,13-dien-1-säure,

(5Z, 11α,13E,15α)-9-Oxo-11,15-dihydroxy-16-methyl-23-tert-butoxy-carbonyl-21,22,23-tri-homo-prosta-5,13-dien-1-säure,
(5Z,11α, 13E, 15α)-9-Oxo-11,15-dihydroxy-16,16-dimethyl-23-tert-butoxy-carbonyl-21,22,23-tri-homo-prosta-5,13-dien-1-säure,
(5Z, 11α,13E, 15α)-9-Oxo-11,15-dihydroxy-19-tert-butoxy-carbonyl-20-nor-prosta-5,13-dien-1-säure,
(5Z, 11α, 13E, 15α)-9-Oxo-11,15-dihydroxy-15β-methyl-19-tert-butoxy-carbonyl-20-nor-prosta-5,13-dien-1-säure,
(5Z, 11α, 13E, 15α)-9-Oxo-11,15-dihydroxy-16-methyl-19-tert-butoxy-carbonyl-20-nor-prosta-5,13-dien-1-säure,

(5Z, 11α, 13E, 15α)-9-Oxo-11,15-dihydroxy-16,16-dimethyl-19-tert-butoxy-carbonyl-20-nor-prosta-5,13-dien-1-säure,
(5Z, 11α, 13E, 15α)-9-Oxo-11,15-dihydroxy-19-iso-propoxycarbonyl-20-nor-prosta-5,13-dien-1-säure,
(5Z, 11α, 13E, 15α)-9-Oxo-11,15-dihydroxy-16,16-dimethyl-19-iso-propoxycarbonyl-20-nor-prosta-5,13-dien-1-säure,
(5Z, 11α, 13E, 15α)-9-Oxo-11,15-dihydroxy-19-carboxy-20-nor-prosta-5,13-dien-1-säure,
(5Z, 11α, 13E, 15α)-9-Oxo-11,15-dihydroxy-16,16-dimethyl-19-carboxy-20-nor-presta-5,13-dien-1-säure,
(5Z, 11α, 13E, 15α)-9-Oxo-11,15-dihydroxy-21-cyano-21-homo-prosta-5,13-dien-1-säure,
(5Z, 11α, 13E, 15α)-9-Oxo-11,15-dihydroxy-15β-methyl-21-cyano-21-homo-prosta-5,13-dien-1-säure,
(5Z, 11α, 13E, 15α)-9-Oxo-11,15-dihydroxy-20-cyano-prosta-5,13-dien-1-säure,
(5Z, 11α, 13E, 15α)-9-Oxo-11,15-dihydroxy-16-methyl-20-cyano-prosta-5,13-dien-1-säure,
(5Z, 11α, 13E, 15α)-9-Oxo-11,15-dihydroxy-16,16-dimethyl-20-cyano-prosta-5,13-dien-1-säure,
(5Z, 11α, 13E, 15α)-9-Oxo-11,15-dihydroxy-19-cyano-20-nor-prosta-5,13-dien-1-säure,
(5Z, 11α, 13E, 15α)-9-Oxo-11,15-dihydroxy-16-methyl-19-cyano-20-nor-prosta-5,13-dien-1-säure,
(5Z, 11α, 13E, 15α)-9-Oxo-11,15-dihydroxy-16,16-dimethyl-19-cyano-20-nor-prosta-5,13-dien-1-säure,
(5Z, 11α, 13E, 15α)-9-Oxo-11,15-dihydroxy-18-cyano-19,20-di-nor-prosta-5,13-dien-1-säure,
(5Z, 11α, 13E, 15α)-9-Oxo-11,15-dihydroxy-16-äthyl-18-cyano-19,20-di-nor-prosta-5,13-dien-1-säure,
(5Z, 11α, 13E, 15α)-9-Oxo-11,15-dihydroxy-16,16-dimethyl-18-cyano-19,20-di-nor-prosta-5,13-dien-1-säure,
(5Z, 11α, 13E, 15α)-9-Oxo-11,15-dihydroxy-18-cyano-20-nor-prosta-5,13-dien-1-säure,
(5Z, 11α, 13E, 15α)-9-Oxo-11,15-dihydroxy-17-cyano-18,19,20-tri-nor-prosta-5,13-dien-1-säure,
(5Z,11α, 13E, 15α)-9-Oxo-11,15-dihydroxy-21-piperidinocarbonyl-21-homo-prosta-5,13-dien-1-säure,

(5Z, 11α, 13E, 15α)-9-Oxo-11,15-dihydroxy-15β-methyl-21-piperidinocarbonyl-21-homo-prosta-5,13-dien-1-säure,

(5Z, 11α, 13E, 15α)-9-Oxo-11,15-dihydroxy-16-methyl-21-piperidinocarbonyl-21-homo-prosta-5,13-dien-1-säure,

(5Z, 11α, 13E, 15α)-9-Oxo-11,15-dihydroxy-16,16-dimethyl-21-piperidinocarbonyl-21-homo-prosta-5,13-dien-1-säure.

(5Z, 11α, 13E, 15α)-9-Oxo-11,15-dihydroxy-17-piperidinocarbonyl-18,19,20-tri-nor-prosta-5,13-dien-1-säure,

(5Z, 11α, 13E, 15α)-9-Oxo-11,15-dihydroxy-16,16-dimethyl-17-piperidinocarbonyl-18,19,20-tri-nor-prosta-5,13-dien-1-säure,

(5Z, 11α, 13E, 15α)-9-Oxo-11,15-dihydroxy-21-dimethylaminocarbonyl-21-homo-prosta-5,13-dien-1-säure,

(5Z, 11α, 13E, 15α)-9-Oxo-11,15-dihydroxy-17-dimethylaminocarbonyl-18,19,20-tri-nor-prosta-5,13-dien-1-säure,

(5Z, 11α, 13E, 15α)-9-Oxo-11,15-dihydroxy-16,16-dimethyl-17-dimethylaminocarbonyl-18,19,20-tri-nor-prosta-5,13-dien-1-säure,

(5Z, 11α, 13E, 15α)-9-Oxo-11,15-dihydroxy-17-(4-methyl-$\Delta^3$-piperideinocarbonyl)-18,19,20-tri-nor-prosta-5,13-dien-1-säure,

(5Z, 11α, 13E, 15α)-9-Oxo-11,15-hydroxy-17-pyrrolidinocarbonyl-18,19,20-tri-nor-prosta-5,13-dien-1-säure,

(5Z, 11α, 13E, 15α)-9-Oxo-11,15-dihydroxy-17-morpholinocarbonyl-18,19,20-tri-nor-prosta-5,13-dien-1-säure,

ihre 15β-Diastereomeren beziehungsweise die nach bekannten Verfahren hergestellten entsprechenden Ester.

In einer weiteren Stufe des erfindungsgemäßen Verfahrens wird entsprechend Reaktionsschema II die Verbindung der allgemeinen Formel (Ib) mit einer Säure behandelt.

Als Säuren eignen sich anorganische Säuren wie Salzsäure, Schwefelsäure, Phosphorsäure, vorzugsweise sei genannt Salzsäure, organische Säuren wie Sulfonsäuren, beispielsweise Methansulfonsäure, Benzolsulfonsäure, p-Toluolsulfonsäure, vorzugsweise p-Toluolsulfonsäure.

Als Verdünnungsmittel kommen solche Lösungsmittel infrage, die mit Wasser mischbar sind und gegenüber den eingesetzten Säuren stabil sind. Es seien beispielsweise genannt: Tetrahydrofuran oder Dioxan.

Als Lösungsmittel eignen sich weiter niedere Alkohole wie Methanol, Aethanol, vorzugsweise Methanol, die bei Abwesenheit von Wasser gegebenenfalls die Ester der Verbindungen der allgemeinen Formel (Ic) liefern.

Die Zusammensetzung des Gemisches ist nicht kritisch, jedoch soll die Verbindung (Ib) darin löslich sein.

Die verwendete Säuremenge richtet sich nach dem pH-Bereich. Der pH-Bereich soll 1 bis 3,5 - vorzugsweise 1,5 bis 2 betragen. Die Temperatur liegt zwischen +10°C und +35°C, vorzugsweise zwischen +20°C und +30°C. Die Reaktionsdauer ist von der Temperatur und dem pH abhängig und liegt zwischen 3 Stunden und 4 Tagen.

Weiterhin lassen sich nach einer Verfahrensvariante Verbindungen der allgemeinen Formel (Ic) direkt aus Verbindungen der allgemeinen Formel (XI) gemäß Reaktionsschema II durch Behandlung mit Säuren herstellen.

Als neue gemäß Reaktionsschema II erhaltene Wirkstoffe seien beispielhaft genannt:

(5Z,13E,15α)-9-Oxo-15-hydroxy-23-tert-butoxycarbonyl-21,22,23-tri-homo-prosta-5,10,13-trien-1-säure,
(5Z,13E,15α)-9-Oxo-15-hydroxy-16-methyl-23-tert-butoxycarbonyl-21,22,23-tri-homo-prosta-5,10,13-trien-1-säure,
(5Z,13E,15α)-9-Oxo-15-hydroxy-16,16-dimethyl-23-tert-butoxycarbonyl-21,22,23-tri-homo-prosta-5,10,13-trien-1-säure,
(5Z,13E,15α)-9-Oxo-15-hydroxy-19-tert-butoxycarbonyl-20-nor-prosta-5,10,13-trien-1-säure,

(5Z, 13E, 15α)-9-Oxo-15-hydroxy-15β-methyl-19-tert-butoxycarbo=nyl-20-nor-prosta-5,10,13-trien-1-säure,
(5Z, 13E, 15α)-9-Oxo-15-hydroxy-16-methyl-19-tert-butoxycarbo=nyl-20-nor-prosta-5,10,13-trien-1-säure,
(5Z, 13E, 15α)-9-Oxo-15-hydroxy-16,16-dimethyl-19-tert-butoxy=carbonyl-20-nor-prosta-5,10,13-trien-1-säure,
(5Z, 13E, 15α)-9-Oxo-15-hydroxy-19-iso-propoxycarbonyl-20-nor-prosta-5,10,13-trien-1-säure,
(5Z, 13E, 15α)-9-Oxo-15-hydroxy-16,16-dimethyl-19-iso-prop=oxycarbonyl-20-nor-prosta-5,10,13-trien-1-säure,
(5Z, 13E, 15α)-9-Oxo-15-hydroxy-19-carboxy-20-nor-prosta-5,10,13-trien-1-säure,
(5Z, 13E, 15α)-9-Oxo-15-hydroxy-16,16-dimethyl-19-carboxy-20-nor-prosta-5,10,13-trien-1-säure,
(5Z, 13E, 15α)-9-Oxo-15-hydroxy-21-cyano-21-homo-prosta-5,10,13-trien-1-säure,
(5Z, 13E, 15α)-9-Oxo-15-hydroxy-15β-methyl-21-cyano-21-homo-prosta-5,10,13-trien-1-säure,
(5Z, 13E, 15α)-9-Oxo-15-hydroxy-20-cyano-prosta-5,10,13-trien-1-säure,
(5Z, 13E, 15α)-9-Oxo-15-hydroxy-16-methyl-20-cyano-prosta-5,10,13-trien-1-säure,
(5Z, 13E, 15α)-9-Oxo-15-hydroxy-16,16-dimethyl-20-cyano-prosta-5,10,13-trien-1-säure,
(5Z, 13E, 15α)-9-Oxo-15-hydroxy-19-cyano-20-nor-prosta-5,10,13-trien-1-säure,
(5Z, 13E, 15α)-9-Oxo-15-hydroxy-16-methyl-19-cyano-20-nor-prosta-5,10,13-trien-1-säure,
(5Z, 13E, 15α)-9-Oxo-15-hydroxy-16,16-dimethyl-19-cyano-20-nor-prosta-5,10,13-trien-1-säure,
(5Z, 13E, 15α)-9-Oxo-15-hydroxy-18-cyano-19,20-di-nor-prosta-5,10,13-trien-1-säure,
(5Z, 13E, 15α)-9-Oxo-15-hydroxy-16-äthyl-18-cyano-19,20-di-nor-prosta-5,10,13-trien-1-säure,
(5Z, 13E, 15α)-9-Oxo-15-hydroxy-16,16-dimethyl-18-cyano-19,20-di-nor-prosta-5,10,13-trien-1-säure,

(5Z, 13E, 15α)-9-Oxo-15-hydroxy-18-cyano-20-nor-prosta-5,10,13-trien-1-säure,
(5Z, 13E, 15α)-9-Oxo-15-hydroxy-17-cyano-18,19,20-tri-nor-prosta-5,10,13-trien-1-säure,
(5Z, 13E, 15α)-9-Oxo-15-hydroxy-21-piperidinocarbonyl-21-homo-prosta-5,10,13-trien-1-säure,
(5Z, 13E, 15α)-9-Oxo-15-hydroxy-15β-methyl-21-piperidinocarbonyl-21-homo-prosta-5,10,13-trien-1-säure,
(5Z, 13E, 15α)-9-Oxo-15-hydroxy-16-methyl-21-piperidino-carbonyl-21-homo-prosta-5,10,13-trien-1-säure,
(5Z, 13E, 15α)-9-Oxo-15-hydroxy-16,16-dimethyl-21-piperidinocarbonyl-21-homo-prosta-5,10,13-trien-1-säure,
(5Z, 13E, 15α)-9-Oxo-15-hydroxy-17-piperidinocarbonyl-18,19,20-tri-nor-prosta-5,10,13-trien-säure,
(5Z, 13E, 15α)-9-Oxo-15-hydroxy-16,16-dimethyl-17-piperidinocarbonyl-18,19,20-tri-nor-prosta-5,10,13-trien-1-säure
(5Z, 13E, 15α)-9-Oxo-15-hydroxy-21-dimethylaminocarbonyl-21-homo-prosta-5,10,13-trien-1-säure,
(5Z, 13E, 15α)-9-Oxo-15-hydroxy-17-dimethylaminocarbonyl-18,19,20-tri-nor-prosta-5,10,13-trien-1-säure,
(5Z, 13E, 15α)-9-Oxo-15-hydroxy-16,16-dimethyl-17-dimethylaminocarbonyl-18,19,20-tri-nor-prosta-5,10,13-trien-1-säure,
(5Z, 13E, 15α)-9-Oxo-15-hydroxy-17-(4-methyl-$\Delta^3$-piperideinocarbonyl)-18,19,20-tri-nor-prosta-5,10,13-trien-1-säure,
(5Z, 13E, 15α)-9-Oxo-15-hydroxy-17-pyrrolidinocarbonyl-18,19,20-tri-nor-prosta-5,10,13-trien-1-säure,
(5Z, 13E, 15α)-9-Oxo-15-hydroxy-17-morpholinocarbonyl-18,19,20-tri-nor-prosta-5,10,13-trien-1-säure,

ihre 15β-Diastereomeren beziehungsweise die nach bekannten Verfahren hergestellten entsprechenden Ester.

Entsprechend dem Reaktionsschema II erhält man in einer weiteren Stufe des erfindungsgemäßen Verfahrens die Verbindungen der allgemeinen Formel (Id), wenn man die Verbindungen der allgemeinen Formel (Ib) mit Basen behandelt.

Als Basen eignen sich beispielsweise Alkali- und Erdalkalihydroxide wie Natriumhydroxid, Kaliumhydroxid, Bariumhydroxid, vorzugsweise Kaliumhydroxid, Alkali- und Erdalkalialkoholate wie Natriummethylat, Natriumäthylat, Kalium-tert-butylat, Magnesiumäthylat, vorzugsweise Natriummethylat, aliphatische und cycloaliphatische Amine und Amidine wie Triäthylamin und 1,8-Diazabicyclo/5,4,0/undec-7-en (DBU), vorzugsweise DBU.

Die Reaktion wird in einem Lösungsmittel durchgeführt, Als Lösungsmittel eignen sich beispielsweise Wasser, niedrige aliphatische Alkohole, insbesondere Methanol und Aethanol.

Die Temperaturen liegen zwischen 0°C und +40°C, vorzugsweise zwischen +10°C und +30°C.

Die verwendete Basenmenge richtet sich nach dem pH-Bereich. Der pH-Bereich liegt bei 7 bis 12, vorzugsweise bei 9 bis 11. Die Reaktionsdauer ist von der Temperatur und vom pH abhängig und liegt zwischen 1 und 5 Stunden.

Als neue Wirkstoffe erhalten gemäß Reaktionsschema II seien beispielhaft genannt:

(5Z, 13E, 15α)-9-Oxo-15-hydroxy-19-tert-butoxycarbonyl-20-nor-prosta-5,8,13-trien-1-säure,
(5Z, 13E, 15α)-9-Oxo-15-hydroxy-15-methyl-19-tert-butoxycarbonyl--20-nor-prosta-5,8,13-trien-1-säure,
(5Z, 13E, 15α)-9-Oxo-15-hydroxy-16,16-dimethyl-19-cyano-20-nor-prosta-5,8,13-trien-1-säure,

(5Z, 13E, 15α)-9-Oxo-15-hydroxy-18-cyano-19,20-dinor-prosta-5,8,13-trien-1-säure,
(5Z, 13E, 15α)-9-Oxo-15-hydroxy-21-piperidinocarbonyl-21-homo-prosta-5,8,13-trien-1-säure,
(5Z, 13E, 15α)-9-Oxo-15-hydroxy-17-piperidinocarbonyl-18,19,20-tri-nor-prosta-5,8,13-trien-1-säure,
(5Z, 13E, 15α)-9-Oxo-15-hydroxy-17-(4-methyl-$\Delta^3$-piperideino-carbonyl)-18,19,20-tri-nor-prosta-5,8,13-trien-1-säure,
(5Z, 13E, 15α)-9-Oxo-15-hydroxy-17-morpholinocarbonyl-18,19,20-tri-nor-prosta-5,8,13-trien-1-säure,

ihre 15β-Diastereomeren beziehungsweise die nach bekannten Verfahren hergestellten entsprechenden Ester.

Die erfindungsgemäßen neuen Prostaglandin-Analoga der allgemeinen Formel (I) haben im Vergleich zu den natürlichen Prostaglandinen differenzierte pharmakologische Wirkungen. Sie sind wertvolle Pharmaka, da sie in der Wirkung spezifischer sind, das heißt, ein engeres biologisches Spektrum besitzen, geringere unerwünschte Nebenwirkungen verursachen, in der erwünschten Wirkung länger anhalten.

Sie eignen sich beispielsweise zur Behandlung von Asthma, zur Herabsetzung einer übermäßigen Magensaftsekretion, als Blutdrucksenker, Antithrombotika oder als Diuretika, als Arzneimittel zur Einleitung von Geburten oder auch als Kontraceptiva zur Anwendung beim Menschen als auch zur Brunstsynchronisation bei verschiedenen Tierarten, beispielsweise Pferden, Rindern oder Schweinen, schließlich auch zur Beeinflussung der Arteriosklerose.

Die neuen erfindungsgemäßen Verbindungen können je nach Verwendungsart beispielsweise intravenös, intramuskulär, subkutan, oral, intravaginal verabreicht werden.
Als Zubereitungsform kommen die üblichen galenischen Applikationsformen infrage, beispielsweise Infusions- oder Injektionslösungen, Tabletten, Cremes, Emulsionen, Suppositorien oder Aerosole.

Die neuen erfindungsgemäßen Verbindungen können als freie Säuren, als Ester oder in Form ihrer physiologisch unbedenklichen anorganischen oder organischen Salze angewendet werden.

Als Salze kommen beispielsweise infrage:

Alkalisalze, Triäthylammonium-, Benzylammonium-, Morpholin-, Triäthanolamin-Salze.

<u>Beispiel 1</u>

2-Oxa-3-oxo-6-(7-tert-butoxycarbonyl-3-oxo-hepten-1-yl)-7-(p-phenylbenzoyloxy)-bicyclo[3,3,0]octan

Zu 33,2 Gewichtsteilen 6-tert-Butoxycarbonyl-2-oxo-hexanphosphonsäuredimethylester in 1000 Volumenteilen wasserfreiem Tetrahydrofuran läßt man unter Inertgas 42,1 Volumenteile Butyllithium (2,2-molare Lösung in Hexan)bei 25°C einfließen. Man rührt 15 Min. und gibt darauf 29,0 Gewichtsteile (∓) 2-Oxa-3-oxo-6-formyl-7-(p-phenylbenzoyloxy)-bicyclo[3,3,0]octan in 430 Volumenteilen wasserfreiem Dimethoxyäthan zu, rührt 1 Stunde bei 25°C, neutralisiert mit Essigsäure, dampft die Lösungsmittel im Vakuum ab, nimmt in Chloroform auf, wäscht mit gesättigter wäßriger Natriumhydrogencarbonat-Lösung und gesättigter wäßriger Natriumchlorid-Lösung, trocknet und engt im Vakuum ein. Der Rückstand wird an Kieselgel mit Cyclohexan und Aceton (2:1) chromatographiert. Man erhält 28,7 Gewichtsteile 2-Oxa-3-oxo-6-(7-tert-butoxycarbonyl-3-oxo-hepten-1-yl)-7-(p-phenylbenzoyloxy)-bicyclo[3,3,0]octan.

(Ausbeute: 65% der Theorie). Fp = 98-100°C

Analyse: $C_{32}H_{36}O_7$ (Molekulargewicht 532,6)

| | | | |
|---|---|---|---|
| Berechnet: | C 72,16 | H 6,82 | O 21,02 |
| Gefunden: | 72,3 | 7,0 | 20,7 |

Massenspektrum: m/e = 278 ($M^+$-$C_4H_8$ und 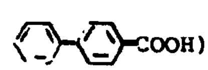)

$^1$H-NMR ($CDCl_3$): δ 6,18 (Dublett), 6,60 (Dublett), 6,78 (Dublett) und 1,43 (Singulett) ppm.

**Beispiele 2-31**

Man setzt 1 Mol eines Aldehyds der allgemeinen Formel (II) mit 1,05 bis 1,3 Mol eines Phosphonesters der allgemeinen Formel (III), der vorher mit 1,05 bis 1,15 Mol einer Base, gegebenenfalls Butyllithium versetzt wurde, in der in Beispiel 1 beschriebenen Weise um und erhält die in Tabelle 1 aufgeführten Reaktionsprodukte.

**Tabelle 1: Hergestellte Verbindungen der allgemeinen Formel (IV)**

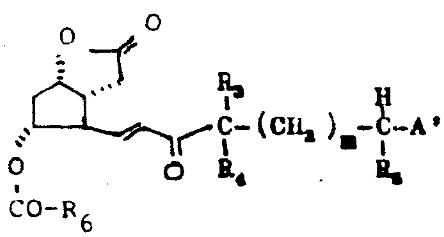

(IV)

| Beispiel Nr. | $R_3$ | $R_4$ | $R_5$ | $R_6$ | m | A' | Ausbeute in % d.Theorie |
|---|---|---|---|---|---|---|---|
| 2 | H | H | H | 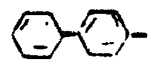 | 6 | $CO_2C(CH_3)_3$ | 64 |
| 3 | $CH_3$ | H | H | 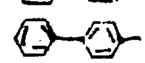 | 6 | $CO_2C(CH_3)_3$ | 70 |
| 4 | $CH_3$ | $CH_3$ | H | 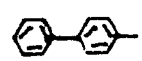 | 6 | $CO_2C(CH_3)_3$ | 69 |
| 5 | $CH_3$ | H | H | 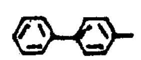 | 2 | $CO_2C(CH_3)_3$ | 68 |
| 6 | $CH_3$ | $CH_3$ | H | 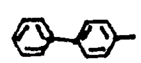 | 2 | $CO_2C(CH_3)_3$ | 65 |
| 7 | H | H | H | 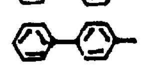 | 2 | $CO_2CH(CH_3)_2$ | 73 |
| 8 | $CH_3$ | $CH_3$ | H | 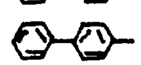 | 2 | $CO_2CH(CH_3)_2$ | 68 |
| 9 | H | H | H | 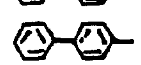 | 4 | CN | 70 |
| 10 | H | H | H | 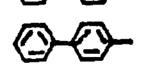 | 3 | CN | 74 |
| 11 | $CH_3$ | H | H | 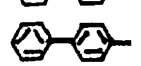 | 3 | CN | 65 |
| 12 | $CH_3$ | $CH_3$ | H | 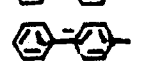 | 3 | CN | 68 |
| 13 | H | H | H | 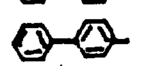 | 2 | CN | 74 |
| 14 | $CH_3$ | H | H | 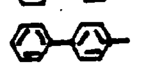 | 2 | CN | 71 |
| 15 | $CH_3$ | $CH_3$ | H | 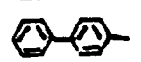 | 2 | CN | 65 |
| 16 | H | H | H | 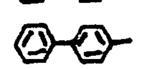 | 1 | CN | 67 |

Tabelle 1 (Fortsetzung)

| Beispiel Nr. | $R_3$ | $R_4$ | $R_5$ | $R_6$ | m | A' | Ausbeute in % d.Theorie |
|---|---|---|---|---|---|---|---|
| 17 | $C_2H_5$ | H | H | 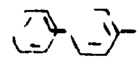 | 1 | CN | 68 |
| 18 | $CH_3$ | $CH_3$ | H | 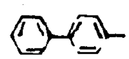 | 1 | CN | 65 |
| 19 | H | H | $CH_3$ | 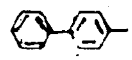 | 1 | CN | 72 |
| 20 | H | H | H | 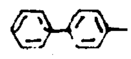 | 0 | CN | 73 |
| 21 | H | H | H | 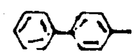 | 4 | 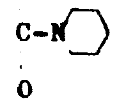 | 65 |
| 22 | $CH_3$ | H | H | 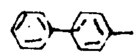 | 4 | 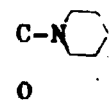 | 71 |
| 23 | $CH_3$ | $CH_3$ | H | 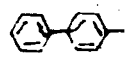 | 4 | 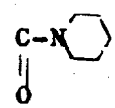 | 64 |
| 24 | H | H | H | 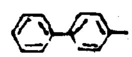 | 0 | 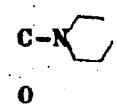 | 73 |
| 25 | $CH_3$ | $CH_3$ | H | 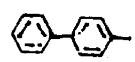 | 0 | 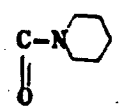 | 64 |
| 26 | H | H | H | 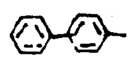 | 4 | 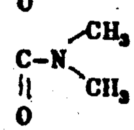 | 71 |
| 27 | H | H | H | 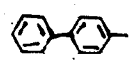 | 0 | 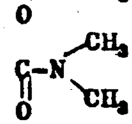 | 75 |
| 28 | $CH_3$ | $CH_3$ | H | 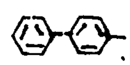 | 0 | 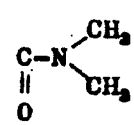 | 72 |
| 29 | H | H | H | 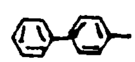 | 0 | 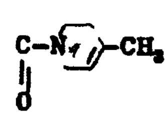 | 63 |
| 30 | H | H | H | 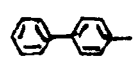 | 0 | 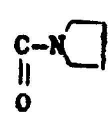 | 74 |
| 31 | H | H | H | 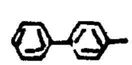 | 0 | 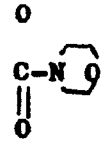 | 73 |

**Beispiel 32**

2-Oxa-3-oxo-6-(9-piperidinocarbonyl-3-oxo-nonen-1-yl)-7-(p-phenyl-benzoyloxy)-bicyclo[3,3,0]octan

Eine Lösung von 110 Gewichtsteilen 8-Piperidinocarbonyl-2-oxo-octanphosphonsäuredimethylester in 1340 Volumenteilen wasserfreiem Dimethoxyäthan wird bei 0 bis +5°C unter Inertgas mit 34,7 Gewichtsteilen Kalium-tert-butylat versetzt, anschließend 15 Minuten bei 20°C gerührt und dann bei 15 bis 20°C mit einer Suspension von 93,5 Gewichtsteilen (∓) 6-Formyl-7-p-phenylbenzoyloxy-3-oxo-2-oxa-bicyclo[3,3,0]octan in 800 Volumenteilen wasserfreiem Dimethoxyäthan versetzt. Man rührt 2 Stunden bei 25°C, versetzt anschließend das Reaktionsgemisch unter Kühlung mit 40 Volumenteilen Eisessig, entfernt das Lösungsmittel im Vakuum bei 40°C, nimmt den Abdampfrückstand in Chloroform auf, saugt vom Niederschlag ab, wäscht das Filtrat nacheinander mit gesättigter Natriumhydrogencarbonatlösung und halbgesättigter Natriumchloridlösung, trocknet die organische Phase mit Natriumsulfat und entfernt das Lösungsmittel im Vakuum. Der Abdampfrückstand wird mit Aether festgerieben, abgesaugt, mit Aether gewaschen und getrocknet. Man erhält 106 Gewichtsteile (Ausbeute 70% der Theorie) 2-Oxa-3-oxo-6-(9-piperidinocarbonyl-3-oxo-nonen-1-yl)-7-(p-phenylbenzoyloxy-bicyclo[3,3,0]-octan.

$^1$H-NMR ($CDCl_3$) $\delta$ = 6,17 (Dublett), 6,59 (Dublett), 6,82 (Dublett) und 3,2 - 3,7 (Multiplett) ppm.

IR (KBr) $\nu$ = 1770, 1720, 1690, 1630, 970 $cm^{-1}$

**Beispiel 33**

2-Oxa-3-oxo-6-(5-piperidino-carbonyl-3-oxo-penten-1-yl)-7-p-phenyl-benzoyloxy)-bicyclo[3,3,0]octan

Eine Lösung von 55,4 Gewichtsteilen 4-Piperidinocarbonyl-2-oxo-butanphosphonsäuredimethylester in 800 Volumenteilen wasserfreiem Dimethoxyäthan wird bei 0 bis +5°C unter Inertgas mit 5,4 Gewichtsteilen 80%igem Natriumhydrid versetzt, anschließend

30 Minuten bei 20°C gerührt und dann bei 15 bis 20°C mit einer Suspension von 56,0 Gewichtsteilen (∓) 6-Formyl-7-p-phenylbenzoyloxy-3-oxo-2-oxa-bicyclo[3,3,0]octan in 480 Volumenteilen wasserfreiem Dimethoxyäthan versetzt. Man rührt 3 Stunden bei 25°C, versetzt anschließend das Reaktionsgemisch unter Kühlung mit 24 Volumenteilen Eisessig, saugt vom Niederschlag ab, dampft das Filtrat im Vakuum bei 40°C ein, nimmt den Abdampfrückstand in Chloroform auf, wäscht die organische Phase nacheinander mit gesättigter Natriumhydrogencarbonatlösung und halbgesättigter Natriumchloridlösung, trocknet die organische Phase mit Natriumsulfat und entfernt das Lösungsmittel im Vakuum. Der Abdampfrückstand wird aus Aethylacetat und Aether umgelöst. Man erhält 60 Gewichtsteile (73% der Theorie) 2-Oxa-3-oxo-6-(5-piperidinocarbonyl-3-oxo-penten-yl)-7-(p-phenyl-benzoyloxy)-bicyclo[3,3,0]octan vom Schmelzpunkt 131-132°C.

Beispiel 34

2-Oxa-3-oxo-6-(7-tert-butoxycarbonyl-3-hydroxy-hepten-1-yl)-7-(p-phenylbenzoyloxy)-bicyclo[3,3,0]octan

Zu 58,8 Gewichtsteilen wasserfreiem Zinkchlorid in 460 Volumenteilen wasserfreiem Dimethoxyäthan gibt man rasch bei 0°C 14,0 Gewichtsteile Natriumborhydrid, rührt 1 Stunde bei 0°C und 8 Stunden bei 20°C. Dazu läßt man bei -20°C 43,8 Gewichtsteile 2-Oxa-3-oxo-6-(7-tert-butoxycarbonyl-3-oxo-hepten-1-yl)-7-(p-phenylbenzoyloxy)-bicyclo[3,3,0]octan in 670 Volumenteilen wasserfreiem Dimethoxyäthan fließen. Man rührt je 1 1/2 Stunden bei -20°C, 0°C und 25°C. Bei 0°C läßt man darauf 76 ml Wasser einfließen, verdünnt mit 1500 Volumenteilen Essigester, saugt ab und dampft das Lösungsmittel im Vakuum ab. Der Rückstand wird in Essigester aufgenommen, mit gesättigter wäßriger Kochsalzlösung gewaschen und getrocknet. Den nach dem Abdampfen des Essigesters erhaltenen Rückstand chromatographiert man an Kieselgel mit Chloroform und Aceton (9:1) und erhält insgesamt 39,9 Gewichtsteile 2-Oxa-3-oxo-6-(7-tert-butoxycarbonyl-3-hydroxy-hepten-1-yl)-7-(p-phenylbenzoyloxy)-bicyclo[3,3,0]-octan (Ausbeute: 93% der Theorie) in Form der aufgetrennten Diastereomerenpaare. Das Verhältnis liegt bei etwa 1:1.

IR: $\nu$ : 3440, 1770 und 1720 $cm^{-1}$.

$^1H$-NMR ($CDCl_3$): $\delta$ = 5,50 - 5,76 (Multiplett) und 1,41 (Singulett) ppm.

**Beispiele 35-64**

Man setzt 1 Mol eines Ketons der allgemeinen Formel (IV) mit ca. 1-2,5 Mol, gegebenenfalls in situ dargestelltem Zinkborhydrid in der in Beispiel 34 beschriebenen Weise um und erhält die in Tabelle 2 aufgeführten Reaktionsprodukte.

**Tabelle 2** Hergestellte Verbindungen der allgemeinen Formel (V)

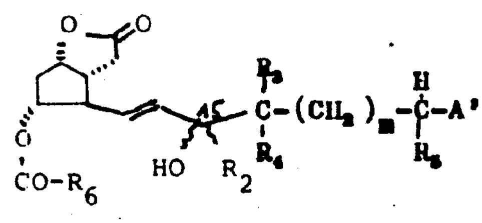

(V)

| Beispiel Nr. | $R_2$ | $R_3$ | $R_4$ | $R_5$ | $R_6$ | m | A' | Ausbeute * i.% d.Th. |
|---|---|---|---|---|---|---|---|---|
| 35 | H | H | H | H | 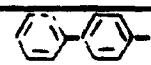 | 6 | $CO_2C(CH_3)_3$ | 97 |
| 36 | H | $CH_3$ | H | H | 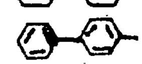 | 6 | $CO_2C(CH_3)_3$ | 95 |
| 37 | H | $CH_3$ | $CH_3$ | H | 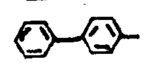 | 6 | $CO_2C(CH_3)_3$ | 99 |
| 38 | H | $CH_3$ | H | H | 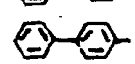 | 2 | $CO_2C(CH_3)_3$ | 96 |
| 39 | H | $CH_3$ | $CH_3$ | H | 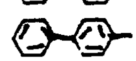 | 2 | $CO_2C(CH_3)_3$ | 94 |
| 40 | H | H | H | H | 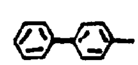 | 2 | $CO_2CH(CH_3)_2$ | 98 |
| 41 | H | $CH_3$ | $CH_3$ | H | 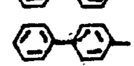 | 2 | $CO_2CH(CH_3)_2$ | 93 |
| 42 | H | H | H | H | 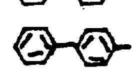 | 4 | CN | 95 |
| 43 | H | H | H | H | 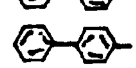 | 3 | CN | 96 |
| 44 | H | $CH_3$ | H | H | 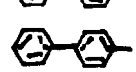 | 3 | CN | 92 |
| 45 | H | $CH_3$ | $CH_3$ | H | 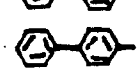 | 3 | CN | 95 |
| 46 | H | H | H | H | 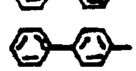 | 2 | CN | 98 |

* Gesamtausbeute der in 15α- bzw. 15β-(PG-Numerierung) aufgetrennten Diastereomeren

0000710



Tabelle 2 (Fortsetzung)

| Beispiel Nr. | $R_2$ | $R_3$ | $R_4$ | $R_5$ | $R_6$ | m | A' | Ausbeute in % d.Theorie |
|---|---|---|---|---|---|---|---|---|
| 47 | H | $CH_3$ | H | H | 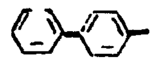 | 2 | CN | 97 |
| 48 | H | $CH_3$ | $CH_3$ | H | 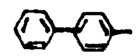 | 2 | CN | 96 |
| 49 | H | H | H | H | 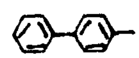 | 1 | CN | 98 |
| 50 | H | $C_2H_5$ | H | H | 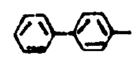 | 1 | CN | 94 |
| 51 | H | $CH_3$ | $CH_3$ | H | 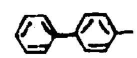 | 1 | CN | 96 |
| 52 | H | H | H | $CH_3$ | 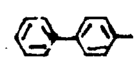 | 1 | CN | 93 |
| 53 | H | H | H | H | 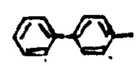 | 0 | CN | 95 |
| 54 | H | H | H | H | 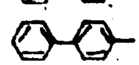 | 4 | 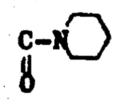 | 98 |
| 55 | H | $CH_3$ | H | H | 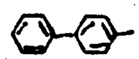 | 4 | 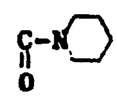 | 96 |
| 56 | H | $CH_3$ | $CH_3$ | H | 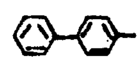 | 4 | 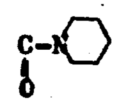 | 95 |
| 57 | H | H | H | H | 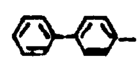 | 0 | 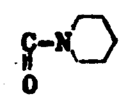 | 97 |
| 58 | H | $CH_3$ | $CH_3$ | H | 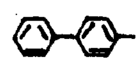 | 0 | 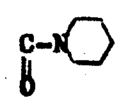 | 93 |
| 59 | H | H | H | H | 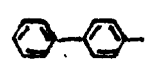 | 4 | 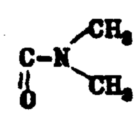 | 98 |
| 60 | H | H | H | H | 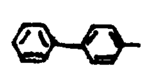 | 0 | 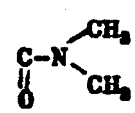 | 98 |
| 61 | H | $CH_3$ | $CH_3$ | H | 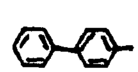 | 0 | 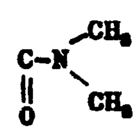 | 96 |

Tabelle 2 (Fortsetzung)

| Beispiel Nr. | $R_2$ | $R_3$ | $R_4$ | $R_5$ | $R_6$ | m | A' | Ausbeute in % d.Theorie * |
|---|---|---|---|---|---|---|---|---|
| 62 | H | H | H | H | 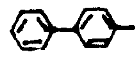 | 0 | 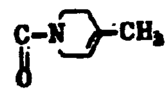 | 95 |
| 63 | H | H | H | H | 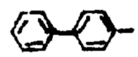 | 0 | 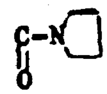 | 97 |
| 64 | H | H | H | H | 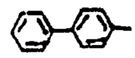 | 0 | 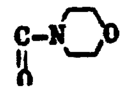 | 91 |

*Gesamtausbeute der in 15α- bzw. 15 β- (PG-Numerierung) aufgetrennten Diastereomeren

**Beispiel 65**

**2-Oxa-3-oxo-6-(6-cyano-3α-hydroxy-hexen-1-yl)-7-(p-phenylbenzoyloxy)-bicyclo [3,3,0] octan**

Zu 22,0 Gewichtsteilen 2-Oxa-3-oxo-6-(6-cyano-3-oxo-hexen-1-yl)-7-(p-phenylbenzoyloxy)-bicyclo[3,3,0]octan in 600 Volumenteilen wasserfreiem Dimethoxyäthan, 100 Volumenteilen wasserfreien Tetrahydrofuran und 600 Volumenteilen wasserfreiem Diäthyläther läßt man unter Inertgas bei -102°C bis -104°C innerhalb von 90 Minuten 300 Volumenteile Kalium-tri-sec.-butylborhydrid (0,5-molare Lösung in Tetrahydrofuran) einfließen und rührt 6 Stunden bei -104°C und 12 Stunden bei -78°C. Bei -70°C läßt man 300 Volumenteile Methanol / 150 Volumenteile ln HCl einfließen. Man dampft ein, nimmt in 1000 Volumenteilen Essigester auf, wäscht mit gesättigter wäßriger Natriumchloridlösung, trocknet und engt im Vakuum ein. Der Rückstand wird an Kieselgel mit Chloroform und Aceton (4:1) chromatographiert. Man erhält 11,6 Gewichtsteile 2-Oxa-3-oxo-6-(6-cyano-3α-hydroxy-hexen-1-yl)-7-(p-phenylbenzoyloxy)-bicyclo[3,3,0]octan

(Ausbeute: 52% der Theorie, $R_f$ = 0,24) und 3,9 Gewichtsteile 2-Oxa-3-oxo-6-(6-cyano-3β-hydroxy-hexen-1-yl)-7-(p-phenylbenzoyloxy)-bicyclo[3,3,0]octan (Ausbeute: 18% der Theorie, $R_f$ = 0,18).

IR: ν : 3420, 2260, 1770 und 1720 $cm^{-1}$

Beispiel 66

2-Oxa-3-oxo-6-(7-tert-butoxycarbonyl-3α-hydroxy-hepten-1-yl)-7-(p-phenylbenzoyloxy)-bicyclo[3,3,0]octan

Man setzt 1 Mol 2-Oxa-3-oxo-6-(7-tert-butoxycarbonyl-3-oxo-hepten-1-yl)-7-(p-phenylbenzoyloxy)-bicyclo[3,3,0]octan mit 1 - 3,5 Mol Kalium-tri-sec.-butylborhydrid in der in Beispiel 65 beschriebenen Weise um und erhält 2-Oxa-3-oxo-6-(7-tert-butoxycarbonyl-3α-hydroxy-hepten-1-yl)-7-(p-phenylbenzoyloxy)-bicyclo[3,3,0]octan (Ausbeute: 53% der Theorie, $R_f$ = 0,45 ) und 2-Oxa-3-oxo-6-(7-tert-butoxycarbonyl-3β-hydroxy-hepten-1-yl)-7-(p-phenylbenzoyloxy)-bicyclo/3,3,0/octan (Ausbeute: 16 % der Theorie, $R_f$ = 0,37).

Beispiel 67

2-Oxa-3-oxo-6-(5-piperidinocarbonyl-3α-hydroxy-penten-1-yl)-7-(p-phenylbenzoyloxy)-bicyclo[3,3,0]octan

26 Gewichtsteile 2-Oxa-3-oxo-6-(5-piperidinocarbonyl-3-oxo-penten-1-yl)-7-(p-phenylbenzoyloxy)-bicyclo[3,3,0]octan werden in 400 Volumteilen wasserfreiem Dimethyloxyäthan bei +60°C gelöst, mit 200 Volumenteilen wasserfreiem Tetrahydrofuran verdünnt und bei -80 bis -85°C rasch mit 120 Volumenteilen einer 0,5 molaren Lithium-perhydro-9b-boraphenalylhydrid-Lösung versetzt. Man rührt anschließend 4 Stunden bei -80° bis -85°C, versetzt dann das Reaktionsgemisch mit 12 Volumenteilen Eisessig, entfernt bei +40°C das Lösungsmittel im Vakuum, nimmt den Abdampfrückstand in Aethylacetat auf, wäscht die organische

Phase mit gesättigter Natriumchloridlösung, trocknet sie über Natriumsulfat und dampft im Vakuum ein. Der Rückstand wird in Chloroform aufgenommen, erneut im Vakuum bei 40°C vom Lösungsmittel befreit und an 2600 Gewichtsteilen Kieselgel mit Chloroform und steigenden Mengen Aceton bis zu einem Chloroform-Aceton-Verhältnis 80:20 chromatographiert. Man erhält 14,5 Gewichtsteile des schneller laufenden 2-Oxa-3-oxo-6-(5-piperidinocarbonyl-3α-hydroxy-penten-1-yl)-7-(p-phenylbenzoyloxy)-bicyclo[3,3,0]octan (Ausbeute: 55% der Theorie). IR: $\nu$ = 1770, 1710, 1610, 975 $cm^{-1}$

Beispiel 68

2-Oxa-3-oxo-6-(7-tert-butoxycarbonyl-3-methyl-3-hydroxy-hepten-1-yl)-7-(p-phenylbenzoyloxy)-bicyclo[3,3,0]octan

1,6 Gewichtsteile 2-Oxa-3-oxo-6-(7-tert-butoxycarbonyl-3-oxo-hepten-1-yl)-7-(p-phenylbenzoyloxy)-bicyclo[3,3,0]octan werden in 30 Volumenteilen wasserfreiem Tetrahydrofuran bei -15°C rasch unter Inertgas mit 2,2 Volumenteilen einer 2,7 molaren Lösung von Methylmagnesiumjodid in Diäthyläther versetzt und 1 Stunde bei -25°C gerührt. Bei -25°C läßt man 8 Volumenteile einer gesättigten wäßrigen Ammoniumchloridlösung einfließen. Man dampft die Lösungsmittel im Vakuum ein, nimmt den Rückstand in Diäthyläther auf, wäscht mit gesättigter wäßriger Natriumchloridlösung, trocknet und chromatographiert den nach dem Abdampfen des Aethers erhaltenen Rückstand an Kieselgel mit 2% Methanol enthaltendem Chloroform. Man erhält 0,96 Gewichtsteile 2-Oxa-3-oxo-6-(7-tert.-butoxycarbonyl-3-methyl-3-hydroxy-hepten-1-yl)-7-(p-phenylbenzoyloxy)-bicyclo[3,3,0]octan (Ausbeute: 58% der Theorie, $R_f$=0,38).

$^1$H-NMR ($CDCl_3$): $\delta$ = 1,43 (Singulett), 1,27 (Singulett) ppm.

Beispiele 69-70

Man setzt 1 Mol eines Ketons der allgemeinen Formel (IV) mit 1-2 Mol Methylmagnesiumjodid in der im Beispiel 68 beschriebenen Weise um und erhält die in Tabelle 3 aufgeführten Reaktionsprodukte.

Tabelle 3: Hergestellte Verbindungen der allgemeinen Formel (V)

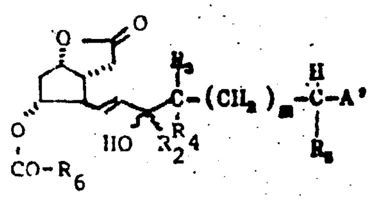

(V)

| Beispiel Nr. | $R_2$ | $R_3$ | $R_4$ | $R_5$ | $R_6$ | m | A' | Ausbeute i.% d.Th. |
|---|---|---|---|---|---|---|---|---|
| 69 | $CH_3$ | H | H | H | 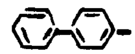 | 4 | CN | 55 |
| 70 | $CH_3$ | H | H | H | 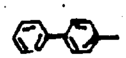 | 4 | 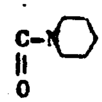 | 61 |

Beispiel 71

2-Oxa-3-oxo-6-(7-tert-butoxycarbonyl-3α-hydroxy-hepten-1-yl)-7-hydroxy-bicyclo[3,3,0]octan

23,0 Gewichtsteile 2-Oxa-3-oxo-6-(7-tert-butoxycarbonyl-3α-hydroxy-hepten-1-yl)-7-(p-phenylbenzoyloxy-)bicyclo[3,3,0]octan und 6,4 Gewichtsteile Kaliumcarbonat rührt man 90 Minuten in 180 Volumenteilen Methanol. Man neutralisiert mit 1n HCl, dampft die Lösungsmittel im Vakuum ab und extrahiert den Rückstand mit Essigester. Die Essigesterphase wird mit gesättigter wäßriger Natriumchloridlösung gewaschen und getrocknet. Der nach dem Abdampfen des Essigesters verbleibende Rückstand wird an Kieselgel mit 10% Methanol enthaltendem Chloroform chroma-

tographiert. Man erhält 14,0 Gewichtsteile 2-Oxa-3-oxo-6-(7-tert-butoxycarbonyl-3α-hydroxy-hepten-1-yl)-7-hydroxy-bicyclo [3,3,0]octan (Ausbeute: 92% der Theorie).

IR: ν : 3350, 1760 und 1721 $cm^{-1}$

$^1$H-NMR ($CDCl_3$): δ = 5,40 - 5,68 (Multiplett) und 1,44 (Singulett) ppm.

<u>Beispiele 72 - 104</u>

Man setzt 1 Mol eines p-Phenylbenzoesäureesters der allgemeinen Formel (V) in der in Beispiel 71 beschriebenen Weise mit 1 - 1,1 Mol Kaliumcarbonat um und erhält die in Tabelle 4 beschriebenen Reaktionsprodukte.

<u>Tabelle 4:</u> Hergestellte Verbindungen der allgemeinen Formel (VI)

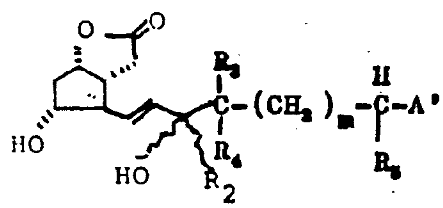

(VI)

| Beispiel Nr. | $R_2$ | $R_3$ | $R_4$ | $R_5$ | m | A' | Ausbeute in % d.Theorie |
|---|---|---|---|---|---|---|---|
| 72 | H | H | H | H | 6 | $CO_2C(CH_3)_3$ | 85 |
| 73 | H | $CH_3$ | H | H | 6 | $CO_2C(CH_3)_3$ | 91 |
| 74 | H | $CH_3$ | $CH_3$ | H | 6 | $CO_2C(CH_3)_3$ | 86 |
| 75 | $CH_3$ | H | H | H | 2 | $CO_2C(CH_3)_3$ | 83 |
| 76 | H | $CH_3$ | H | H | 2 | $CO_2C(CH_3)_3$ | 90 |
| 77 | H | $CH_3$ | $CH_3$ | H | 2 | $CO_2C(CH_3)_3$ | 91 |
| 78 | H | H | H | H | 2 | $CO_2CH(CH_3)_2$ | 84 |
| 79 | H | $CH_3$ | $CH_3$ | H | 2 | $CO_2CH(CH_3)_2$ | 87 |
| 80 | H | H | H | H | 4 | CN | 83 |
| 81 | $CH_3$ | H | H | H | 4 | CN | 86 |

Tabelle 4 (Fortsetzung)

| Beispiel Nr. | $R_2$ | $R_3$ | $R_4$ | $R_5$ | m | A' | Ausbeute in % d.Theorie |
|---|---|---|---|---|---|---|---|
| 82 | H | H | H | H | 3 | CN | 86 |
| 83 | H | $CH_3$ | H | H | 3 | CN | 84 |
| 84 | H | $CH_3$ | $CH_3$ | H | 3 | CN | 82 |
| 85 | H | H | H | H | 2 | CN | 81 |
| 86 | H | $CH_3$ | H | H | 2 | CN | 88 |
| 87 | H | $CH_3$ | $CH_3$ | H | 2 | CN | 87 |
| 88 | H | H | H | H | 1 | CN | 90 |
| 89 | H | $C_2H_5$ | H | H | 1 | CN | 85 |
| 90 | H | $CH_3$ | $CH_3$ | H | 1 | CN | 92 |
| 91 | H | H | H | $CH_3$ | 1 | CN | 90 |
| 92 | H | H | H | H | 0 | CN | 88 |
| 93 | H | H | H | H | 4 | 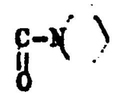 | 82 |
| 94 | $CH_3$ | H | H | H | 4 | 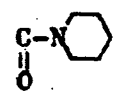 | 83 |
| 95 | H | $CH_3$ | H | H | 4 | 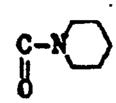 | 87 |
| 96 | H | $CH_3$ | $CH_3$ | H | 4 | 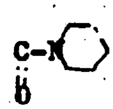 | 93 |
| 97 | H | H | H | H | 0 | 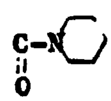 | 88 |
| 98 | H | $CH_3$ | $CH_3$ | H | 0 | 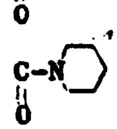 | 85 |
| 99 | H | H | H | H | 4 | 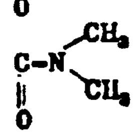 | 92 |
| 100 | H | H | H | H | 0 | 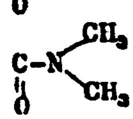 | 84 |

Tabelle 4 (Fortsetzung)

| Beispiel Nr. | $R_2$ | $R_3$ | $R_4$ | $R_5$ | m | A' | Ausbeute in % d.Theorie |
|---|---|---|---|---|---|---|---|
| 101 | H | $CH_3$ | $CH_3$ | H | 0 | 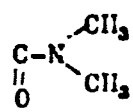 | 90 |
| 102 | H | H | H | H | 0 | 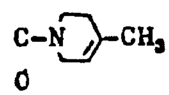 | 85 |
| 103 | H | H | H | H | 0 | 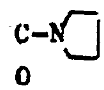 | 83 |
| 104 | H | H | H | H | 0 | 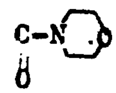 | 86 |

Beispiel 105

2-Oxa-3-oxo-6-(7-tert-butoxycarbonyl-3α-tetrahydropyranyloxy-hepten-1-yl)-7-tetrahydropyranyloxy-bicyclo[3,3,0]octan

Zu 57,6 Gewichtsteilen 2-Oxa-3-oxo-6-(7-tert-butoxycarbonyl-3α-hydroxy-hepten-1-yl)-7-hydroxy-bicyclo[3,3,0]octan und 65,0 Gewichtsteilen Dihydropyran in 600 Volumenteilen Methylenchlorid läßt man 29 Volumenteile einer 2%igen p-Toluolsulfonsäure in Tetrahydrofuran einfließen. Man rührt 1 Stunde bei 25°C, gibt darauf 16 Volumenteile Pyridin und 2000 Volumenteile Essigester zu. Man wäscht mit gesättigter wäßriger Natriumhydrogencarbonatlösung und gesättigter wäßriger Natriumchloridlösung, trocknet, dampft die Lösungsmittel im Vakuum ein und chromatographiert den Rückstand an Kieselgel mit Cyclohexan und Aceton (2:1). Man erhält 65,4 Gewichtsteile 2-Oxa-3-oxo-6-(7-tert-butoxycarbonyl-3α-tetrahydropyranyloxy-hepten-1-yl)-7-tetrahydropyranyloxy-bicyclo[3,3,0]octan (Ausbeute: 77% der Theorie).

IR: ν : 1726 und 1778 $cm^{-1}$.

**Beispiele 106 - 138**

Man setzt 1 Mol eines Diols der allgemeinen Formel (VI) in der in Beispiel 105 beschriebenen Weise mit 1,5 - 5 Mol Dihydropyran um und erhält die in der Tabelle 5 beschriebenen Reaktionsprodukte.

**Tabelle 5:** Hergestellte Verbindungen der allgemeinen Formel (VII)

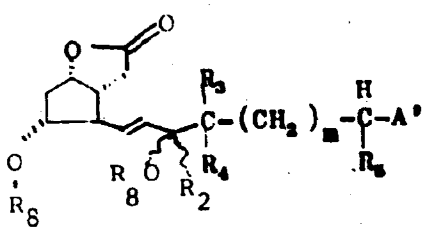

(VII)

| Beispiel Nr. | $R_2$ | $R_3$ | $R_4$ | $R_5$ | $R_8$ | m | A' | Ausbeute in % d.Theorie |
|---|---|---|---|---|---|---|---|---|
| 106 | H | H | H | H | THP | 6 | $CO_2C(CH_3)_3$ | 80 |
| 107 | H | $CH_3$ | H | H | THP | 6 | $CO_2C(CH_3)_3$ | 78 |
| 108 | H | $CH_3$ | $CH_3$ | H | THP | 6 | $CO_2C(CH_3)_3$ | 79 |
| 109 | $CH_3$ | H | H | H | THP | 2 | $CO_2C(CH_3)_3$ | 81 |
| 110 | H | $CH_3$ | H | H | THP | 2 | $CO_2C(CH_3)_3$ | 82 |
| 111 | H | $CH_3$ | $CH_3$ | H | THP | 2 | $CO_2C(CH_3)_3$ | 78 |
| 112 | H | H | H | H | THP | 2 | $CO_2CH(CH_3)_2$ | 77 |
| 113 | H | $CH_3$ | $CH_3$ | H | THP | 2 | $CO_2CH(CH_3)_2$ | 80 |
| 114 | H | H | H | H | THP | 4 | CN | 81 |
| 115 | $CH_3$ | H | H | H | THP | 4 | CN | 84 |
| 116 | H | H | H | H | THP | 3 | CN | 82 |

THP = 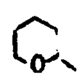

- 44 -

Tabelle 5 (Fortsetzung)

| Beispiel Nr. | $R_2$ | $R_3$ | $R_4$ | $R_5$ | $R_6$ | m | A' | Ausbeute in % d.Theorie |
|---|---|---|---|---|---|---|---|---|
| 117 | H | $CH_3$ | H | H | THP | 3 | CN | 78 |
| 118 | H | $CH_3$ | $CH_3$ | H | THP | 3 | CN | 79 |
| 119 | H | H | H | H | THP | 2 | CN | 77 |
| 120 | H | $CH_3$ | H | H | THP | 2 | CN | 77 |
| 121 | H | $CH_3$ | $CH_3$ | H | THP | 2 | CN | 79 |
| 122 | H | H | H | H | THP | 1 | CN | 82 |
| 123 | H | $C_2H_5$ | H | H | THP | 1 | CN | 82 |
| 124 | H | $CH_3$ | $CH_3$ | H | THP | 1 | CN | 85 |
| 125 | H | H | H | $CH_3$ | THP | 1 | CN | 71 |
| 126 | H | H | H | H | THP | 0 | CN | 75 |
| 127 | H | H | H | H | THP | 4 | 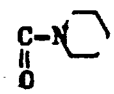 | 73 |
| 128 | $CH_3$ | H | H | H | THP | 4 | 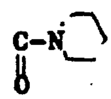 | 80 |
| 129 | H | $CH_3$ | H | H | THP | 4 | 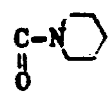 | 81 |
| 130 | H | $CH_3$ | $CH_3$ | H | THP | 4 | 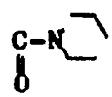 | 84 |
| 131 | H | H | H | H | THP | 0 | 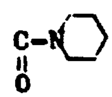 | 77 |
| 132 | H | $CH_3$ | $CH_3$ | H | THP | 0 | 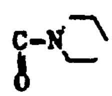 | 76 |
| 133 | H | H | H | H | THP | 4 | 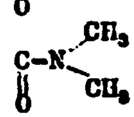 | 85 |
| 134 | H | H | H | H | THP | 0 | 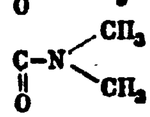 | 83 |
| 135 | H | $CH_3$ | $CH_3$ | H | THP | 0 | 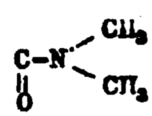 | 82 |

THP = 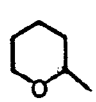

Tabelle 5 (Fortsetzung)

| Beispiel Nr. | $R_2$ | $R_3$ | $R_4$ | $R_8$ | $R_9$ | m | A' | Ausbeute in % d.Theorie |
|---|---|---|---|---|---|---|---|---|
| 136 | H | H | H | H | THP | 0 | 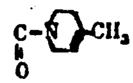 | 84 |
| 137 | H | H | H | H | THP | 0 | 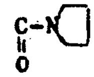 | 85 |
| 138 | H | H | H | H | THP | 0 | 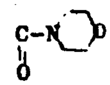 | 81 |

THP = 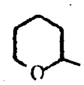

**Beispiel 135**

**2-Oxa-3-oxo-6-(5-piperidinocarbonyl-3α-(tert-butyl-dimethyl-silyloxy)-penten-1-yl)-7-(tert-butyl-dimethyl-silyloxy)-bi=cyclo[3,3,0]octan**

2,45 Gewichtsteile 2-Oxa-3-oxo-6-(5-piperidinocarbonyl-3α-hydroxy-penten-1-yl)-7-hydroxy-bicyclo[3,3,0]octan (Schmelz=punkt 124-125°C) werden zusammen mit 2,97 Gewichtsteilen Imid=azol, 3,2 Volumenteilen Dimethylformamid und 2,2 Gewichtsteilen tert-Butyl-dimethyl-silylchlorid 10 Stunden bei 37°C unter Inertgas gerührt. Man läßt 12 Stunden bei 25°C stehen, ent=fernt das Lösungsmittel bei 40°C im Vakuum, verdünnt den Abdampfrückstand mit Methylenchlorid, wäscht die organische Phase mit Wasser, trocknet sie über Natriumsulfat und entfernt das Lösungsmittel im Vakuum. Der Abdampfrückstand (3,7 Gewichts=teile) wird an 220 Gewichtsteilen Kieselgel mit Chloroform chromatographiert. Man erhält 3,3 Gewichtsteile 2-Oxa-3-oxo-6-(5-piperidinocarbonyl-3α-(tert-butyl-dimethyl-silyloxy)-pen=ten-1-yl)-7-(tert-butyl-dimethyl-silyloxy)-bicyclo[3,3,0]octan (Ausbeute: 80% der Theorie).

IR: $\nu$: = 1770, 1640, 980, 840, 780 $cm^{-1}$.

$^1H$-NMR ($CDCl_3$): $\delta$ = 5,30-5,50 (Multiplett), 4,68-5,00 (Multiplett), 3,70 - 4,30 (Multiplett), 3,15 - 3,67 (Multiplett), 0,83 (Singulett) und 0,85 (Singulett) ppm.

Beispiel 140

2-Oxa-3-hydroxy-6-(7-tert-butoxycarbonyl)-3α-tetrahydropyranyloxy-hepten-1-yl)-7-tetrahydropyranyloxy-bicyclo[3,3,0]octan.

Zu 49,6 Gewichtsteilen 2-Oxa-3-oxo-6-(7-tert-butoxycarbonyl-3α-tetrahydropyranyloxy-hepten-1-yl)-7-tetrahydropyranyloxy-bicyclo[3,3,0]octan in 600 Volumenteilen wasserfreiem Toluol läßt man unter Inertgasatmosphäre 116 Volumenteile einer 70 %igen Lösung von Natrium-bis-(2-methoxyäthoxy)-di-hydridoaluminat in Benzol verdünnt mit 500 Volumenteilen wasserfreiem Toluol bei -70°C bis -78°C einfließen. Man rührt 6 Stunden in diesem Temperaturintervall. Bei -70°C läßt man darauf 380 Volumenteile Wasser und 380 Volumenteile Methanol einfließen, verdünnt mit 400 Volumenteilen Toluol, versetzt mit 1200 Volumenteilen gesättigter wäßriger Natriumchloridlösung, extrahiert mit Essigester, wäscht mit gesättigter wäßriger Natriumchloridlösung, trocknet und dampft die Lösungsmittel im Vakuum ab. Man erhält 49,6 Gewichtsteile 2-Oxa-3-hydroxy-6-(7-tert-butoxycarbonyl-3α-tetrahydropyranyloxy-hepten-1-yl)-7-tetrahydropyranyloxy-bicyclo[3,3,0]octan.

(Ausbeute: 100 % der Theorie).

IR: ν: 3360 und 1726 $cm^{-1}$.

Beispiele 141 - 173

**Man setzt 1 Mol eines** Lactons der allgemeinen Formel (VII) in **der in Beispiel 140 beschriebenen Weise mit 1 - 4,5 Mol Natrium bis-(2-methoxyäthoxy)-di-hydrido-aluminat um und erhält die in Tabelle 6 beschriebenen Reaktionsprodukte.**

**Tabelle 6: Hergestellte Verbindungen der allgemeinen Formel (VIII)**

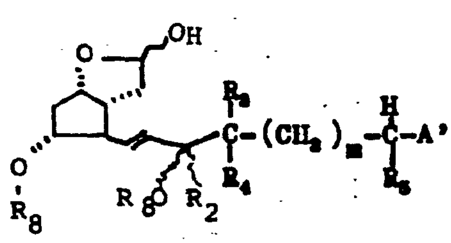

**(VIII)**

| Beispiel Nr. | $R_2$ | $R_3$ | $R_4$ | $R_5$ | $R_6$ | m | A' | Ausbeute in % d.Theorie |
|---|---|---|---|---|---|---|---|---|
| 141 | H | H | H | H | THP | 6 | $CO_2C(CH_3)_3$ | 98 |
| 142 | H | $CH_3$ | H | H | THP | 6 | $CO_2C(CH_3)_3$ | 98 |
| 143 | H | $CH_3$ | $CH_3$ | H | THP | 6 | $CO_2C(CH_3)_3$ | 99 |
| 144 | $CH_3$ | H | H | H | THP | 2 | $CO_2C(CH_3)_3$ | 100 |
| 145 | H | $CH_3$ | H | H | THP | 2 | $CO_2C(CH_3)_3$ | 99 |
| 146 | H | $CH_3$ | $CH_3$ | H | THP | 2 | $CO_2C(CH_3)_3$ | 98 |
| 147 | H | H | H | H | THP | 2 | $CO_2CH(CH_3)_2$ | 100 |
| 148 | H | $CH_3$ | $CH_3$ | H | THP | 2 | $CO_2CH(CH_3)_2$ | 99 |
| 149 | H | H | H | H | THP | 4 | CN | 97 |
| 150 | $CH_3$ | H | H | H | THP | 4 | CN | 96 |
| 151 | H | H | H | H | THP | 3 | CN | 100 |
| 152 | H | $CH_3$ | H | H | THP | 3 | CN | 99 |
| 153 | H | $CH_3$ | $CH_3$ | H | THP | 3 | CN | 99 |
| 154 | H | H | H | H | THP | 2 | CN | 98 |
| 155 | H | $CH_3$ | H | H | THP | 2 | CN | 99 |
| 156 | H | $CH_3$ | $CH_3$ | H | THP | 2 | CN | 100 |
| 157 | H | H | H | H | THP | 1 | CN | 98 |
| 158 | H | $C_2H_5$ | H | H | THP | 1 | CN | 97 |
| 159 | H | $CH_3$ | $CH_3$ | H | THP | 1 | CN | 99 |
| 160 | H | H | H | $CH_3$ | THP | 1 | CN | 95 |
| 161 | H | H | H | H | THP | 0 | CN | 98 |
| 162 | H | H | H | H | THP | 4 | 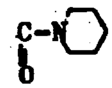 | 97 |
| 163 | $CH_3$ | H | H | H | THP | 4 | 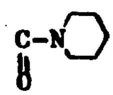 | 96 |
| 164 | H | $CH_3$ | H | H | THP | 4 | 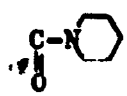 | 98 |
| 165 | H | $CH_3$ | $CH_3$ | H | THP | 4 | 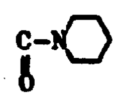 | 99 |
| 166 | H | H | H | H | THP | 0 | 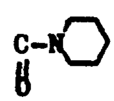 | 99 |

Tabelle 6 (Fortsetzung)

| Beispiel Nr. | $R_2$ | $R_3$ | $R_4$ | $R_5$ | $R_6$ | m | A' | Ausbeute in % d.Theorie |
|---|---|---|---|---|---|---|---|---|
| 167 | H | $CH_3$ | $CH_3$ | H | THP | 0 | 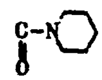 | 97 |
| 168 | H | H | H | H | THP | 4 | 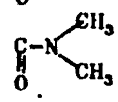 | 95 |
| 169 | H | H | H | H | THP | 0 | 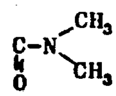 | 93 |
| 170 | H | $CH_3$ | $CH_3$ | H | THP | 0 | 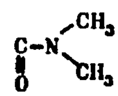 | 94 |
| 171 | H | H | H | H | THP | 0 | 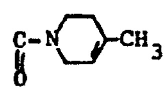 | 89 |
| 172 | H | H | H | H | THP | 0 | 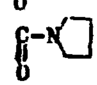 | 95 |
| 173 | H | H | H | H | THP | 0 | 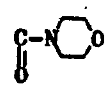 | 96 |

**Beispiel 174**

(5Z, 9α, 11α, 13E, 15α)-9-Hydroxy-11,15-di -tetrahydropyranyl= oxy-19-tert-butoxycarbonyl-20-nor-prosta-5,13-dien-1-säure

Unter Inertgas läßt man bei 5°C bis 10°C 19,8 Gewichtsteile Triphenyl-4-carboxy-butylphosphoniumbromid in 200 Volumenteilen wasserfreiem Dimethoxyäthan zu 16,6 Gewichtsteilen Natrium-bis-(trimethylsilyl)-amid in 150 Volumenteilen wasserfreiem Dimethoxyäthan einfließen und rührt 1 Stunde bei 25°C. Darauf läßt man bei 5°C bis 10°C 8,4 Gewichtsteile 2-Oxa-3-hydroxy-6-(7-tert-butoxycarbonyl-3α-tetrahydropyranyloxy-hepten-1-yl)-7-tetrahydropyranyloxy-bicyclo[3,3,0]octan in 100 Volumenteilen wasserfreiem Dimethoxyäthan einfließen und rührt noch 3 Stunden

bei 25°C. Man gibt 3 Volumenteile Wasser zu, dampft das Lö= sungsmittel im Vakuum ab, verteilt den Rückstand zwischen 140 Volumenteilen Wasser und 4x 200 Volumenteilen Diäthyläther. Die wäßrige Phase wird mit Oxalsäure unter Kühlung auf pH 4 eingestellt und mit Pentan und Diäthyläther (1:1) extrahiert. Man trocknet und erhält nach dem Abdampfen des Lösungsmittels 7,8 Gewichtsteile (5Z, 9α, 11α, 13E, 15α)-9-Hydroxy-11,15-di-tetrahydropyranyloxy-19-tert-butoxycarbonyl-20-nor-prosta-5,13-dien-1-säure (Ausbeute 81% der Theorie).

IR: ν : 1725 und 1710 $cm^{-1}$.

Beispiele 175 - 207

Man setzt 1 Mol eines Lactols der allgemeinen Formel (VIII) in der in Beispiel 174 beschriebenen Weise mit 1,3 bis 2,8 Mol Phosphoniumsalz der allgemeinen Formel (IX) um und erhält die in der Tabelle 7 beschriebenen Reaktionsprodukte.

Tabelle 7: Hergestellte Verbindungen der allgemeinen Formel (X)

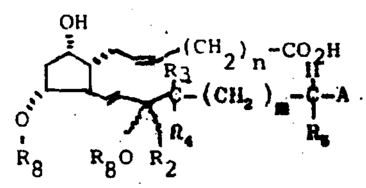

(X)

| Beispiel Nr. | $R_2$ | $R_3$ | $R_4$ | $R_5$ | $R_8$ | m | n | A | Ausbeute in % d.Theorie |
|---|---|---|---|---|---|---|---|---|---|
| 175 | H | H | H | H | THP | 6 | 3 | $CO_2C(CH_3)_3$ | 77 |
| 176 | H | $CH_3$ | H | H | THP | 6 | 3 | $CO_2C(CH_3)_3$ | 75 |
| 177 | H | $CH_3$ | $CH_3$ | H | THP | 6 | 3 | $CO_2C(CH_3)_3$ | 73 |
| 178 | $CH_3$ | H | H | H | THP | 2 | 3 | $CO_2C(CH_3)_3$ | 78 |
| 179 | H | $CH_3$ | H | H | THP | 2 | 3 | $CO_2C(CH_3)_3$ | 76 |
| 180 | H | $CH_3$ | $CH_3$ | H | THP | 2 | 3 | $CO_2C(CH_3)_3$ | 73 |
| 181 | H | H | H | H | THP | 2 | 3 | $CO_2CH(CH_3)_2$ | 74 |
| 182 | H | $CH_3$ | $CH_3$ | H | THP | 2 | 3 | $CO_2CH(CH_3)_2$ | 75 |
| 183 | H | H | H | H | THP | 4 | 3 | CN | 80 |

Tabelle 7 (Fortsetzung)

| Beispiel Nr. | $R_2$ | $R_3$ | $R_4$ | $R_5$ | $R_8$ | m | n | A | Ausbeute in % d. Theorie |
|---|---|---|---|---|---|---|---|---|---|
| 184 | $CH_3$ | H | H | H | THP | 4 | 3 | CN | 81 |
| 185 | H | H | H | H | THP | 3 | 3 | CN | 78 |
| 186 | H | $CH_3$ | H | H | THP | 3 | 3 | CN | 77 |
| 187 | H | $CH_3$ | $CH_3$ | H | THP | 3 | 3 | CN | 80 |
| 188 | H | H | H | H | THP | 2 | 3 | CN | 82 |
| 189 | H | $CH_3$ | H | H | THP | 2 | 3 | CN | 83 |
| 190 | H | $CH_3$ | $CH_3$ | H | THP | 2 | 3 | CN | 83 |
| 191 | H | H | H | H | THP | 1 | 3 | CN | 79 |
| 192 | H | $C_2H_5$ | H | H | THP | 1 | 3 | CN | 77 |
| 193 | H | $CH_3$ | $CH_3$ | H | THP | 1 | 3 | CN | 75 |
| 194 | H | H | H | $CH_3$ | THP | 1 | 3 | CN | 80 |
| 195 | H | H | H | H | THP | 0 | 3 | CN | 78 |
| 196 | H | H | H | H | THP | 4 | 3 | 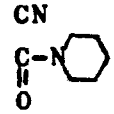 | 75 |
| 197 | $CH_3$ | H | H | H | THP | 4 | 3 | 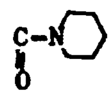 | 77 |
| 198 | H | $CH_3$ | H | H | THP | 4 | 3 | 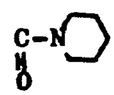 | 70 |
| 199 | H | $CH_3$ | $CH_3$ | H | THP | 4 | 3 | 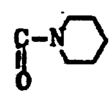 | 78 |
| 200 | H | H | H | H | THP | 0 | 3 | 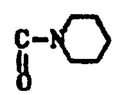 | 77 |
| 201 | H | $CH_3$ | $CH_3$ | H | THP | 0 | 3 | 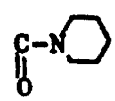 | 74 |
| 202 | H | H | H | H | THP | 4 | 3 | 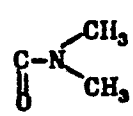 | 80 |
| 203 | H | H | H | H | THP | 0 | 3 | 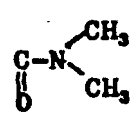 | 82 |

und erhält nach dem Abdampfen des Lösungsmittels 6,1 Gewichtsteile (5Z, 9α, 11α, 13E, 15α)-9-Hydroxy-11,15-di-tetrahydropyranyloxy-20-nor-prosta-5,13-dien-1,19-disäure (Ausbeute : 58% der Theorie).
IR: ν : 1710 $cm^{-1}$.

Beispiel 209

(5Z, 9α, 11α, 13E, 15α)-9-Hydroxy-11,15-di-tetrahydropyranyloxy-16,16-dimethyl-20-nor-prosta-5,13-dien-1,19-di-säure

Man setzt 1 Mol 2-Oxa-3-hydroxy-6-(7-tert-butoxy carbonyl-3α-tetrahydropyranyloxy-4,4-dimethyl-hepten-1-yl)-7-tetrahydropyranyloxy-bicyclo[3,3,0]octan mit dem aus 2,8 Mol Triphenyl-4-carboxy-butyl-phosphoniumbromid und Natriumhydrid in Dimethylsulfoxid erzeugten Produkt in der in Beispiel 208 beschriebenen Weise um und erhält (5Z, 9α, 11α, 13E, 15α)-9-Hydroxy-11,15-di-tetrahydropyranyloxy-16,16-dimethyl-20-nor-prosta-5,13-dien-1,19-disäure (Ausbeute: 60 % d. Theorie).
IR: ν: 1710, 1080, 1027 und 979 $cm^{-1}$.

Beispiel 210

(5Z, 9α, 11α, 13E, 15α)-9,11,15-Trihydroxy-19-tert-butoxycarbonyl-20-nor-prosta-5,13-dien-1-säure

29,0 Gewichtsteile (5Z, 9α, 11α, 13E, 15α)-9-Hydroxy-11,15-di-tetrahydropyranyloxy-19-tert-butoxycarbonyl-20-nor-5,13-dien-1-säure erwärmt man 6 Stunden in 290 Volumenteilen Essigsäure, Wasser und Tetrahydrofuran (2:1:0,3) auf 55°C. Man dampf die Lösungsmittel im Vakuum ab, chromatographiert den Rückstand an Kieselgel mit Chloroform,Essigsäure und Tetrahydrofuran (10:1:2) und erhält 10,9 Gewichtsteile (5Z, 9α, 11α, 13E, 15α)-9,11,15-Trihydroxy-19-tert-butoxycarbonyl-20-nor-prosta-5,13-dien-1-säure (Ausbeute: 52% der Theorie).

Tabelle 7 (Fortsetzung)

| Beispiel-Nr. | $R_2$ | $R_3$ | $R_4$ | $R_5$ | $R_8$ | m | n | A | Ausbeute in % d. Theorie |
|---|---|---|---|---|---|---|---|---|---|
| 204 | H | $CH_3$ | $CH_3$ | H | THP | 0 | 3 | 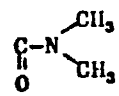 | 78 |
| 205 | H | H | H | H | THP | 0 | 3 | 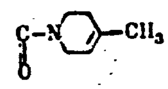 | 68 |
| 206 | H | H | H | H | THP | 0 | 3 | 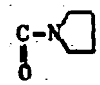 | 73 |
| 207 | H | H | H | H | THP | 0 | 3 | 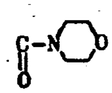 | 78 |

**Beispiel 208**

(5Z, 9α, 11α, 13E, 15α)-9-hydroxy-11,15-di-tetrahydropyranyloxy-20-nor-prosta-5,13-dien-1,19-di-säure

2,55 Gewichtsteile Natriumhydrid gibt man zu 55 Volumenteilen wasserfreiem Dimethylsulfoxid unter Inertgas und erwärmt 2 Stunden auf 60 bis 70°C. Dazu läßt man bei 15°C bis 16°C 23,4 Gewichtsteile Triphenyl-4-carboxy-butylphosphonium-bromid in 100 Volumenteilen wasserfreiem Dimethylsulfoxid einfließen. Darauf läßt man 10,0 Gewichtsteile 2-Oxa-3-hydroxy-6-(7-tert-butoxycarbonyl-3α-tetrahydropyranyloxy-hepten-1-yl)-7-tetrahydropyranyloxy-bicyclo[3,3,0]octan in 85 Volumenteilen wasserfreiem Dimethylsulfoxid bei 15°C bis 16°C einfließen und rührt noch 2 Stunden bei 25°C. Nach Zugabe von 3,5 Volumenteien Wasser wird das Lösungsmittel im Vakuum abdestilliert und der Rückstand zwischen 170 Volumenteilen Wasser und 4x 240 Volumenteilen Diäthyläther verteilt. Die wäßrige Phase wird mit Oxalsäure auf pH 4 unter Kühlung eingestellt und mit Pentan und Diäthyläther (1:1) extrahiert. Man trocknet

IR: $\nu$: 1726, 1710 und 977 $cm^{-1}$

$^1H$-NMR ($CDCl_3$): $\delta$ = 5,26 - 5,65 (Multiplett) und 1,41 (Singulett) ppm.

Wahlweise läßt sich auch die Diastereomerentrennung (15α, 15β), die in Beispiel 34 auf der Stufe des Allylalkohols (V) beschrieben wurde, durch Chromatographie der $C_{15}$-Diastereomerenpaare von (5Z, 9α, 11α, 13E, 15α/β)-9,11,15-Trihydroxy-19-tert-butoxycarbonyl-20-nor-prosta-5,13-dien-1-säure an Kieselgel beispielsweise mit Essigsäure, Essigester und Methanol (1:90:4) gut durchführen.

Das gilt auch für die in Tabelle 8 aufgeführten Verbindungen.

Beispiele 211-243

Man setzt 1 Mol einer Säure der allgemeinen Formel (X) in der in Beispiel 210 beschriebenen Weise mit Essigsäure, Wasser und Tetrahydrofuran um und erhält die in Tabelle 8 aufgeführten Reaktionsprodukte.

Tabelle 8: Hergestellte Verbindungen der allgemeinen Formel (Ia)

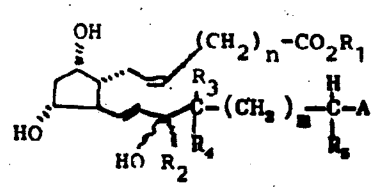

(Ia)

| Beispiel Nr. | $R_1$ | $R_2$ | $R_3$ | $R_4$ | $R_5$ | m | n | A | Ausbeute in % d.Theorie |
|---|---|---|---|---|---|---|---|---|---|
| 211 | H | H | H | H | H | 6 | 3 | $CO_2C(CH_3)_3$ | 59 |
| 212 | H | H | $CH_3$ | H | H | 6 | 3 | $CO_2C(CH_3)_3$ | 60 |
| 213 | H | H | $CH_3$ | $CH_3$ | H | 6 | 3 | $CO_2C(CH_3)_3$ | 55 |
| 214 | H | $CH_3$ | H | H | H | 2 | 3 | $CO_2C(CH_3)_3$ | 57 |

Tabelle 8 (Fortsetzung)

| Beispiel Nr. | $R_1$ | $R_2$ | $R_3$ | $R_4$ | $R_5$ | m | n | A | Ausbeute in % d.Theorie |
|---|---|---|---|---|---|---|---|---|---|
| 215 | H | H | $CH_3$ | H | H | 2 | 3 | $CO_2C(CH_3)_3$ | 63 |
| 216 | H | H | $CH_3$ | $CH_3$ | H | 2 | 3 | $CO_2C(CH_3)_3$ | 60 |
| 217 | H | H | H | H | H | 2 | 3 | $CO_2CH(CH_3)_2$ | 57 |
| 218 | H | H | $CH_3$ | $CH_3$ | H | 2 | 3 | $CO_2CH(CH_3)_2$ | 52 |
| 219 | H | H | H | H | H | 2 | 3 | $CO_2H$ | 85 |
| 220 | H | H | $CH_3$ | $CH_3$ | H | 2 | 3 | $CO_2H$ | 83 |
| 221 | H | H | H | H | H | 4 | 3 | CN | 64 |
| 222 | H | $CH_3$ | H | H | H | 4 | 3 | CN | 62 |
| 223 | H | H | H | H | H | 3 | 3 | CN | 65 |
| 224 | H | H | $CH_3$ | H | H | 3 | 3 | CN | 60 |
| 225 | H | H | $CH_3$ | $CH_3$ | H | 3 | 3 | CN | 61 |
| 226 | H | H | H | H | H | 2 | 3 | CN | 66 |
| 227 | H | H | $CH_3$ | H | H | 2 | 3 | CN | 63 |
| 228 | H | H | $CH_3$ | $CH_3$ | H | 2 | 3 | CN | 68 |
| 229 | H | H | H | H | H | 1 | 3 | CN | 66 |
| 230 | H | H | $C_2H_5$ | H | H | 1 | 3 | CN | 69 |
| 231 | H | H | $CH_3$ | $CH_3$ | H | 1 | 3 | CN | 67 |
| 232 | H | H | H | H | $CH_3$ | 1 | 3 | CN | 65 |
| 233 | H | H | H | H | H | 0 | 3 | CN | 68 |
| 234 | H | H | H | H | H | 4 | 3 | 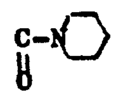 | 70 |
| 235 | H | $CH_3$ | H | H | H | 4 | 3 | 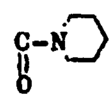 | 71 |
| 236 | H | H | $CH_3$ | H | H | 4 | 3 | 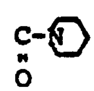 | 68 |
| 237 | H | H | $CH_3$ | $CH_3$ | H | 4 | 3 | 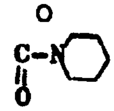 | 66 |
| 238 | H | H | H | H | H | 0 | 3 | 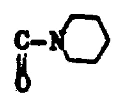 | 69 |
| 239 | H | H | $CH_3$ | $CH_3$ | H | 0 | 3 | 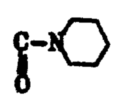 | 68 |

Tabelle 8 (Fortsetzung)

| Beispiel Nr. | $R_1$ | $R_2$ | $R_3$ | $R_4$ | $R_5$ | m | n | A | Ausbeute in % d.Theorie |
|---|---|---|---|---|---|---|---|---|---|
| 240 | H | H | H | H | H | 4 | 3 | 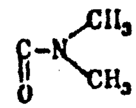 | 63 |
| 241 | H | H | H | H | H | 0 | 3 | 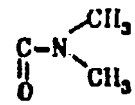 | 62 |
| 242 | H | H | $CH_3$ | $CH_3$ | H | 0 | 3 | 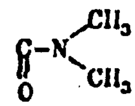 | 58 |
| 243 | H | H | H | H | H | 0 | 3 | 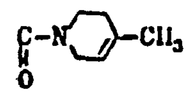 | 56 |
| 244 | H | H | H | H | H | 0 | 3 | 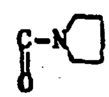 | 63 |
| 245 | H | H | H | H | H | 0 | 3 | 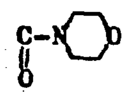 | 62 |

Beispiel 246

(5Z, 11α, 13E, 15α)-9-Oxo-11,15-di-tetrahydropyranyloxy-19-tert-butoxycarbonyl-20-nor-prosta-5,13-dien-1-säure

Zu 7,8 Gewichtsteilen (5Z, 9α, 11α, 13E, 15α)-9-Hydroxy-11.15-di-tetrahydropyranyloxy-19-tert-butoxycarbonyl-20-nor-prosta-5,13-dien-1-säure in 320 Volumenteilen Aceton läßt man bei -20°C 17,8 Volumenteile einer schwefelsauren 2,67 normalen Chromtrioxid lösung in Wasser einfließen und rührt 3 Stunden bei -20°C. Bei -20°C läßt man darauf 49,5 Volumenteile 2-Propanol ein= fließen und stellt nach Zugabe von 17 Volumenteilen Wasser den pH auf 4,8 durch Versetzen mit Natriumhydrogencarbonat ein. Man filtriert ab, dampft das Lösungsmittel im Vakuum ein, nimmt in Essigester auf, trocknet und erhält nach dem Ab= dampfen des Lösungsmittels 7,8 Gewichtsteile (5Z, 11α, 13E, 15α)-9-Oxo-11,15-ditetrahydropyranyloxy-19-tert-butoxycarbo= nyl-20-nor-prosta-5,13-dien-1-säure (Rohausbeute: 100% der Theorie).

$^1$H-NMR ($CDCl_3$): $\delta$ = 5,20-5,76 (Multiplett und 1,41 (Singulett). ppm.

Beispiele 247-279

Man setzt 1 Mol einer Säure der allgemeinen Formel (X) in der in Beispiel 246 beschriebenen Weise mit 2 bis 4 Mol Chromtrioxid um und erhält die in Tabelle 9 beschriebenen Reaktionsprodukte.

Tabelle 9: Hergestellc Verbindungen der allgemeinen Formel (XI

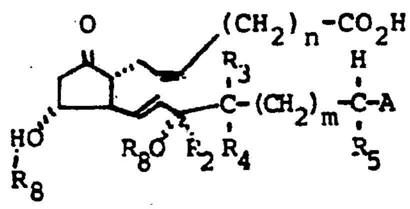

(XI)

| Beispiel Nr. | $R_2$ | $R_3$ | $R_4$ | $R_5$ | $R_8$ | m | n | A | Ausbeute in % d.Theorie |
|---|---|---|---|---|---|---|---|---|---|
| 247 | H | H | H | H | THP | 6 | 3 | $CO_2C(CH_3)_3$ | 98 |
| 248 | H | $CH_3$ | H | H | THP | 6 | 3 | $CO_2C(CH_3)_3$ | 99 |
| 249 | H | $CH_3$ | $CH_3$ | H | THP | 6 | 3 | $CO_2C(CH_3)_3$ | 97 |
| 250 | $CH_3$ | H | H | H | THP | 2 | 3 | $CO_2C(CH_3)_3$ | 100 |
| 251 | H | $CH_3$ | H | H | THP | 2 | 3 | $CO_2C(CH_3)_3$ | 99 |
| 252 | H | $CH_3$ | $CH_3$ | H | THP | 2 | 3 | $CO_2C(CH_3)_3$ | 98 |
| 253 | H | H | H | H | THP | 2 | 3 | $CO_2CH(CH_3)_2$ | 96 |
| 254 | H | $CH_3$ | $CH_3$ | H | THP | 2 | 3 | $CO_2CH(CH_3)_2$ | 96 |
| 255 | H | H | H | H | THP | 4 | 3 | CN | 97 |
| 256 | $CH_3$ | H | H | H | THP | 4 | 3 | CN | 98 |
| 257 | H | H | H | H | THP | 3 | 3 | CN | 96 |
| 258 | H | $CH_3$ | H | H | THP | 3 | 3 | CN | 100 |
| 259 | H | $CH_3$ | $CH_3$ | H | THP | 3 | 3 | CN | 98 |
| 260 | H | H | H | H | THP | 2 | 3 | CN | 93 |
| 261 | H | $CH_3$ | H | H | THP | 2 | 3 | CN | 97 |

* Rohausbeute

Tabelle 9 (Fortsetzung)

| Beispiel Nr. | $R_2$ | $R_3$ | $R_4$ | $R_5$ | $R_6$ | m | n | A | Ausbeute in % d.Theorie* |
|---|---|---|---|---|---|---|---|---|---|
| 262 | H | $CH_3$ | $CH_3$ | H | THP | 2 | 3 | CN | 96 |
| 263 | H | H | H | H | THP | 1 | 3 | CN | 98 |
| 264 | H | $C_2H_5$ | H | H | THP | 1 | 3 | CN | 99 |
| 265 | H | $CH_3$ | $CH_3$ | H | THP | 1 | 3 | CN | 97 |
| 266 | H | H | H | $CH_3$ | THP | 1 | 3 | CN | 96 |
| 267 | H | H | H | H | THP | 0 | 3 | CN | 98 |
| 268 | H | H | H | H | THP | 4 | 3 | 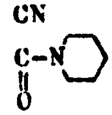 | 95 |
| 269 | $CH_3$ | H | H | H | THP | 4 | 3 | 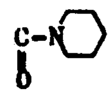 | 99 |
| 270 | H | $CH_3$ | H | H | THP | 4 | 3 | 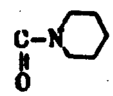 | 97 |
| 271 | H | $CH_3$ | $CH_3$ | H | THP | 4 | 3 | 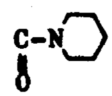 | 98 |
| 272 | H | H | H | H | THP | 0 | 3 | 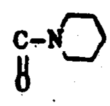 | 100 |
| 273 | H | $CH_3$ | $CH_3$ | H | THP | 0 | 3 | 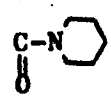 | 99 |
| 274 | H | H | H | H | THP | 4 | 3 | 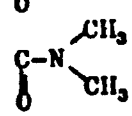 | 98 |
| 275 | H | H | H | H | THP | 0 | 3 | 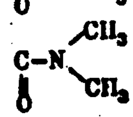 | 97 |
| 276 | H | $CH_3$ | $CH_3$ | H | THP | 0 | 3 | 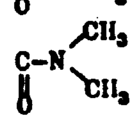 | 99 |
| 277 | H | H | H | H | THP | 0 | 3 | 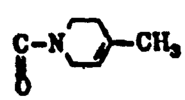 | 89 |
| 278 | H | H | H | H | THP | 0 | 3 | 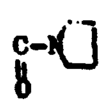 | 93 |
| 279 | H | H | H | H | THP | 0 | 3 | 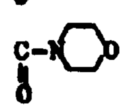 | 92 |

*Rohausbeute

Beispiel 280

(5Z, 11α, 13E, 15α)-9-Oxo-11,15-dihydroxy-19-tert-butoxy-carbonyl-20-nor-prosta-5,13-dien-1-säure

30,9 Gewichtsteile (5Z, 11α, 13E, 15α)-9-Oxa-11,15-di-tetrahydropyranyloxy-19-tert-butoxycarbonyl-20-nor-prosta-5,13-dien-1-säure erwärmt man 4 Stunden in 310 Volumenteilen Essigsäure, Wasser und Tetrahydrofuran (2:1:0,3) auf 42°C. Man versetzt mit 310 Volumenteilen Wasser und destilliert die Lösungsmittel im Vakuum schonend ab. Der Rückstand liefert nach der Chromatographie an Kieselgel mit Chloroform Tetrahydrofuran und Essigsäure (10:2:1) 7,1 Gewichtsteile (5Z, 11α, 13E, 15α)-9-Oxo-11,15-dihydroxy-19-tert-butoxy-carbonyl-20-nor-prosta-5,13-dien-1-säure (Ausbeute: 32% der Theorie).

IR: $\nu$ : 1740 und 1719 $cm^{-1}$

$^1$H-NMR ($CDCl_3$): $\delta$ : 5,50-5,77 (Multiplett), 5,18-5,50 (Multiplett) und 1,43 (Singulett) ppm.

Die Diastereomerentrennung (15α, 15β) läßt sich auch statt auf der Stufe des Allylalkohols (V) (siehe Beispiel 34) auch durch Chromatographie der $C_{15}$-Diastereomerenpaare von (5Z, 11α, 13E, 15α/β)-9-Oxo-11,15-dihydroxy-19-tert-butoxy-carbonyl-20-nor-prosta-5,13-dien-1-säure an Kieselgel mit beispielsweise obigem Gemisch durchführen.

Dies gilt auch für die in der Tabelle 10 aufgeführten Verbindungen.

Beispiele 281 - 315

Man setzt 1 Mol einer Säure der allgemeinen Formel (XI) in der in Beispiel 280 beschriebenen Weise mit Essigsäure, Wasser und Tetrahydrofuran um und erhält die in Tabelle 10 aufgeführten Reaktionsprodukte.

**Tabelle 10:** **Hergestellte Verbindungen der allgemeinen Formel (Ib)**

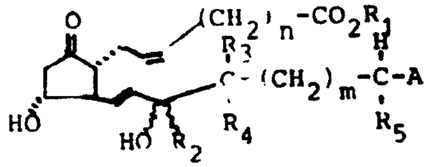

(Ib)

| Beispiel Nr. | $R_1$ | $R_2$ | $R_3$ | $R_4$ | $R_5$ | m | n | A | Ausbeute in % d.Theorie |
|---|---|---|---|---|---|---|---|---|---|
| 281 | H | H | H | H | H | 6 | 3 | $CO_2C(CH_3)_3$ | 40 |
| 282 | H | H | $CH_3$ | H | H | 6 | 3 | $CO_2C(CH_3)_3$ | 43 |
| 283 | H | H | $CH_3$ | $CH_3$ | H | 6 | 3 | $CO_2C(CH_3)_3$ | 38 |
| 284 | H | $CH_3$ | H | H | H | 2 | 3 | $CO_2C(CH_3)_3$ | 35 |
| 285 | H | H | $CH_3$ | H | H | 2 | 3 | $CO_2C(CH_3)_3$ | 42 |
| 286 | H | H | $CH_3$ | $CH_3$ | H | 2 | 3 | $CO_2C(CH_3)_3$ | 44 |
| 287 | H | H | H | H | H | 2 | 3 | $CO_2CH(CH_3)_2$ | 38 |
| 288 | H | H | $CH_3$ | $CH_3$ | H | 2 | 3 | $CO_2CH(CH_3)_2$ | 33 |
| 289 | H | H | H | H | H | 2 | 3 | $CO_2H$ | 36 |
| 290 | H | H | $CH_3$ | $CH_3$ | H | 2 | 3 | $CO_2H$ | 35 |
| 291 | H | H | H | H | H | 4 | 3 | CN | 55 |
| 292 | H | $CH_3$ | H | H | H | 4 | 3 | CN | 52 |
| 293 | H | H | H | H | H | 3 | 3 | CN | 48 |
| 294 | H | H | $CH_3$ | H | H | 3 | 3 | CN | 54 |
| 295 | H | H | $CH_3$ | $CH_3$ | H | 3 | 3 | CN | 49 |
| 296 | H | H | H | H | H | 2 | 3 | CN | 50 |
| 297 | H | H | $CH_3$ | H | H | 2 | 3 | CN | 53 |
| 298 | H | H | $CH_3$ | [illegible] | H | 2 | 3 | CN | 51 |
| 299 | H | H | H | [illegible] | [illegible] | [illegible] | 3 | CN | 55 |
| 300 | H | H | $C_2H_5$ | H | H | [illegible] | 3 | CN | 49 |
| 301 | H | H | $CH_3$ | [illegible] | H | [illegible] | 3 | CN | 55 |
| 302 | H | H | H | [illegible] | [illegible] | [illegible] | 3 | CN | 50 |
| 303 | H | H | H | [illegible] | H | 0 | 3 | CN | 53 |
| 304 | H | H | H | [illegible] | H | 4 | 3 | 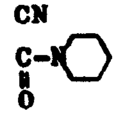 | 35 |

Tabelle 10 (Fortsetzung)

| Beispiel Nr. | $R_1$ | $R_2$ | $R_3$ | $R_4$ | $R_5$ | m | n | A | Ausbeute in % d.Theorie |
|---|---|---|---|---|---|---|---|---|---|
| 305 | H | $CH_3$ | H | H | H | 4 | 3 | 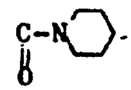 | 31 |
| 306 | H | H | $CH_3$ | H | H | 4 | 3 | 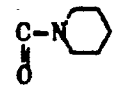 | 34 |
| 307 | H | H | $CH_3$ | $CH_3$ | H | 4 | 3 | 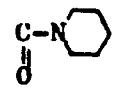 | 32 |
| 308 | H | H | H | H | H | 0 | 3 | 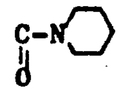 | 30 |
| 309 | H | H | $CH_3$ | $CH_3$ | H | 0 | 3 | 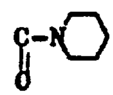 | 28 |
| 310 | H | H | H | H | H | 4 | 3 | 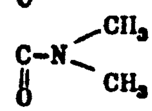 | 37 |
| 311 | H | H | H | H | H | 0 | 3 | 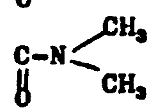 | 30 |
| 312 | H | H | $CH_3$ | $CH_3$ | H | 0 | 3 | 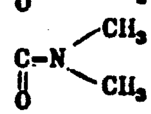 | 34 |
| 313 | H | H | H | H | H | 0 | 3 | 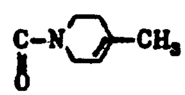 | 31 |
| 314 | H | H | H | H | H | 0 | 3 | 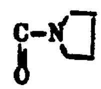 | 29 |
| 315 | H | H | H | H | H | 0 | 3 | 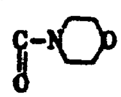 | 30 |

**Beispiel 316**

(5Z, 13E, 15α)-9-Oxo-15-hydroxy-19-tert-butoxycarbonyl-20-nor-prosta-5,10,13-trien-1-säure

2,5 Gewichtsteile (5Z, 11α, 13E, 15α)-9-Oxo-11,15-dihydroxy-19-tert-butoxycarbonyl-20-nor-prosta-5,13-dien-1-säure in 20 Volumenteilen Tetrahydrofuran säuert man mit 1n HCl auf pH 2 an und beläßt den Ansatz 3 Tage bei 25°C. Mit festem Natriumhydrogencarbonat stellt man unter Zusatz von 2 Volumenteilen Wasser auf pH 4 ein, engt im Vakuum ein, nimmt in wenig Essigester auf, trocknet, filtriert und chromatographiert an Kieselgel mit Chloroform, Essigsäure und Tetrahydrofuran (10:2:1). Man erhält 1 Gewichtsteil (5Z, 13E, 15α)-9-Oxo-15-hydroxy-19-tert-butoxycarbonyl-20-nor-prosta-5,10,13-trien-1-säure (Ausbeute: 42% der Theorie).

IR: $\nu$: 1728 und 1711 $cm^{-1}$

$^1$H-NMR ($CDCl_3$): $\delta$ = 7,43 (Dublett), 7,51 (Dublett), 6,12 (Dublett), 6,21 (Dublett), 5,51-5,71 (Multiplett), 5,22-5,51 (Multiplett) und 1,45 (Singulett) ppm.

**Beispiele 317 - 351**

Man setzt 1 Mol einer Säure der allgemeinen Formel (Ib) mit beispielsweise Salzsäure in der in Beispiel 316 beschriebenen Weise um und erhält die in der Tabelle 11 aufgeführten Reaktionsprodukte.

Tabelle 11: **Hergestellte Verbindungen der allgemeinen Formel (Ic)**

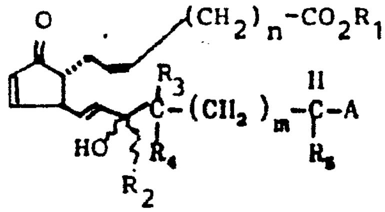

(Ic)

| Beispiel Nr. | $R_1$ | $R_2$ | $R_3$ | $R_4$ | $R_5$ | m | n | A | Ausbeute % d.Theo |
|---|---|---|---|---|---|---|---|---|---|
| 317 | H | H | H | H | H | 6 | 3 | $CO_2C(CH_3)_3$ | 45 |
| 318 | H | H | $CH_3$ | H | H | 6 | 3 | $CO_2C(CH_3)_3$ | 40 |
| 319 | H | H | $CH_3$ | $CH_3$ | H | 6 | 3 | $CO_2C(CH_3)_3$ | 43 |
| 320 | H | $CH_3$ | H | H | H | 2 | 3 | $CO_2C(CH_3)_3$ | 46 |
| 321 | H | H | $CH_3$ | H | H | 2 | 3 | $CO_2C(CH_3)_3$ | 42 |
| 322 | H | H | $CH_3$ | $CH_3$ | H | 2 | 3 | $CO_2C(CH_3)_3$ | 39 |
| 323 | H | H | H | H | H | 2 | 3 | $CO_2CH(CH_3)_2$ | 45 |
| 324 | H | H | $CH_3$ | $CH_3$ | H | 2 | 3 | $CO_2CH(CH_3)_2$ | 43 |
| 325 | H | H | H | H | H | 2 | 3 | $CO_2H$ | 46 |
| 326 | H | H | $CH_3$ | $CH_3$ | H | 2 | 3 | $CO_2H$ | 46 |
| 327 | H | H | H | H | H | 4 | 3 | CN | 38 |
| 328 | H | $CH_3$ | H | H | H | 4 | 3 | CN | 39 |
| 329 | H | H | H | H | H | 3 | 3 | CN | 42 |
| 330 | H | H | $CH_3$ | H | H | 3 | 3 | CN | 42 |
| 331 | H | H | $CH_3$ | $CH_3$ | H | 3 | 3 | CN | 38 |
| 332 | H | H | H | H | H | 2 | 3 | CN | 39 |
| 333 | H | H | $CH_3$ | H | H | 2 | 3 | CN | 43 |
| 334 | H | H | $CH_3$ | $CH_3$ | H | 2 | 3 | CN | 41 |
| 335 | H | H | H | H | H | 1 | 3 | CN | 35 |
| 336 | H | H | $C_2H_5$ | H | H | 1 | 3 | CN | 42 |
| 337 | H | H | $CH_3$ | $CH_3$ | H | 1 | 3 | CN | 38 |
| 338 | H | H | H | H | $CH_3$ | 1 | 3 | CN | 41 |
| 339 | H | H | H | H | H | 0 | 3 | CN | 40 |
| 340 | H | H | H | H | H | 4 | 3 | 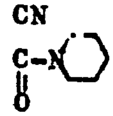 | 35 |

Tabelle 11 ( Fortsetzung )

| Beispiel Nr. | $R_1$ | $R_2$ | $R_3$ | $R_4$ | $R_5$ | m | n | A | Ausbeute in % d.Theorie |
|---|---|---|---|---|---|---|---|---|---|
| 341 | H | $CH_3$ | H | H | H | 4 | 3 | 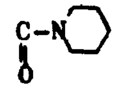 | 37 |
| 342 | H | H | $CH_3$ | H | H | 4 | 3 | 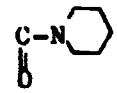 | 31 |
| 343 | H | H | $CH_3$ | $CH_3$ | H | 4 | 3 | 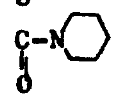 | 32 |
| 344 | H | H | H | H | H | 0 | 3 | 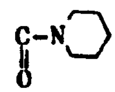 | 38 |
| 345 | H | H | $CH_3$ | $CH_3$ | H | 0 | 3 | 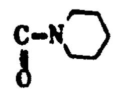 | 36 |
| 346 | H | H | H | H | H | 4 | 3 | 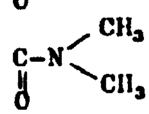 | 30 |
| 347 | H | H | H | H | H | 0 | 3 | 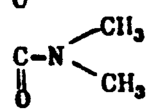 | 32 |
| 348 | H | H | $CH_3$ | $CH_3$ | H | 0 | 3 | 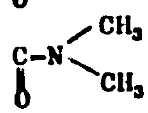 | 37 |
| 349 | H | H | H | H | H | 0 | 3 | 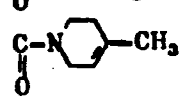 | 31 |
| 350 | H | H | H | H | H | 0 | 3 | 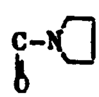 | 30 |
| 351 | H | H | H | H | H | 0 | 3 | 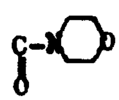 | 32 |

Beispiel 352

(5Z, 13E, 15α)-9-Oxo-15-hydroxy-18-cyano-19,20-dinor-prosta-5,10,13-trien-1-säure-methylester

Zu 1,0 Gewichtsteile (5Z, 13E, 15α)-9-Oxo-15-hydroxy-18-cyano-19,20-dinor-prosta-5,10,13-dien-1-säure in 10 Volumenteilen Tetrahydrofuran und 10 Volumenteilen Methanol gibt man 0,2 Gewichtsteile p-Toluolsulfonsäure. Man beläßt den Ansatz 15 Stunden bei 25°C. Mit festem Natriumhydrogencarbonat stellt man den pH auf 4,5 ein, dampft die Lösungsmittel im Vakuum ab, nimmt in 50 Volumenteilen Diäthyläther auf, filtriert, wäscht mit 10 Volumenteilen gesättigter wäßriger Natriumchlo= ridlösung, trocknet und chromatographiert den nach dem Abdamp= fen des Diäthyläthers erhaltenen Rückstand an Kieselgel mit Chloroform, Tetrahydrofuran und Essigsäure (10:2:1). Man er= hält 0,4 Gewichtsteile (5Z, 13E, 15α)-9-Oxo-15-hydroxy-18-cyano-19,20-dinor-prosta-5,10,13-trien-1-säuremethylester (Ausbeute: 42% der Theorie).

IR: $\nu$ : 3400, 2460, 1730 und 1713 $cm^{-1}$

$^1$H-NMR ($CDCl_3$): $\delta$ = 3,63 (Singulett) ppm.

Beispiel 353

(5Z, 13E, 15α/β-)9-Oxo-15-hydroxy-16-methyl-19-tert-butoxy-carbonyl-20-nor-prosta-5,10,13-trien-1-säure

Zu 1,0 Gewichtsteilen (5Z, 13E, 11α, 15α/15β)-9-Oxo-11,15-di-tetrahydropyranyloxy-16-methyl-19-tert-butoxycarbonyl-20-nor-prosta-5,13-dien-1-säure in 10 Volumenteilen Tetrahydrofuran gibt man 0,2 Volumenteile einer 1-normalen wäßrigen Salzsäure. Man läßt 4 Tage bei 25°C stehen, versetzt dann mit 5 Volumenteilen Wasser und mit festem Natriumhydrogencarbonat bis zum pH 4. Man dampft die Lösungsmittel im Vakuum ab, chromatographiert den Rückstand an Kieselgel mit Chloroform, Tetrahydrofuran, Essigsäure (10:2:0,5). Man erhält 0,2 Gewichtsteile (5Z, 13E, 15α/15β)-9-Oxo-15-hydroxy-16-methyl-19-tert-butoxycarbonyl-

20-nor-prosta-5,10,13-trien-1-säure (Ausbeute: 28% der Theorie).
$^1$H-NMR ($CDCl_3$): $\delta$ = 6,18 (Dublett), 6,23 (Dublett) und 1,43 (Singulett) ppm.

Beispiel 354
(5Z, 13E, 15α)-9-Oxo-15-hydroxy-18-cyano-19,20-dinor-prosta-5,8,13-trien-1-säure

Zu 2,5 Gewichtsteilen (5Z, 11α, 13E, 15α)-9-Oxo-11,15-dihydroxy-18-cyano-19,20-dinor-prosta-5,13-dien-1-säure in 10 Volumenteilen Methanol gibt man pulverisiertes Kaliumhydroxid bis zum pH 10. Man rührt 3 Stunden bei 25°C, säuert mit 10%iger Salzsäure auf pH 3 an, engt im Vakuum ein, nimmt in Essigester auf, filtriert, trocknet und dampft den Essigester im Vakuum ab. Man chromatographiert an Kieselgel mit Chloroform, Tetrahydrofuran und Essigsäure (10:2:1) und erhält 1,9 Gewichtsteile (5Z, 13E, 15α)-9-Oxo-15-hydroxy-18-cyano-19,20-dinor-prosta-5,8,13-trien-1-säure (Ausbeute: 80% der Theorie).

IR: $\nu$ : 2260, 1730, 1705 und 1640 $cm^{-1}$.
$^1$H-NMR ($CDCl_3$): $\delta$ = 5,22 - 5,53 (Multiplett) ppm.

Beispiele 355 - 361
Man setzt 1 Mol einer Säure der allgemeinen Formel (Ib) in der in Beispiel 354 beschriebenen Weise bei einem pH von 9-11 um und erhält die in der Tabelle 12 aufgeführten Reaktionsprodukte.

Tabelle 12 : Hergestellte Verbindungen der allgemeinen Formel (Id)

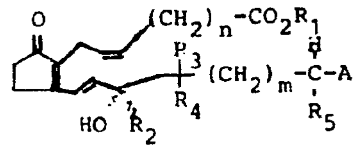

(Id)

| Beispiel Nr. | $R_1$ | $R_2$ | $R_3$ | $R_4$ | $R_5$ | m | n | A | Ausbeute in % d.Theorie |
|---|---|---|---|---|---|---|---|---|---|
| 355 | H | H | H | H | H | 2 | 3 | $CO_2C(CH_3)_3$ | 81 |
| 356 | H | $CH_3$ | H | H | H | 2 | 3 | $CO_2C(CH_3)_3$ | 85 |
| 357 | H | H | $CH_3$ | $CH_3$ | H | 2 | 3 | CN | 83 |
| 358 | H | H | H | H | H | 4 | 3 | 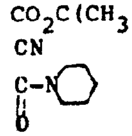 | 84 |
| 359 | H | H | H | H | H | 0 | 3 | 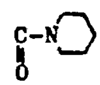 | 82 |
| 360 | H | H | H | H | H | 0 | 3 | 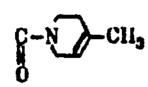 | 79 |
| 361 | H | H | H | H | H | 0 | 3 | 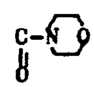 | 77 |

Beispiel 362

(5Z, 9α, 11α, 13E, 15α)-9,11,15-Trihydroxy-19-tert-butoxycarbonyl-20-nor-prosta-5,13-dien-1-säure-methylester

5,0 Gewichtsteile (5Z, 9α, 11α, 13E, 15α)-9,11,15-Trihydroxy-19-tert-butoxycarbonyl-20-nor-prosta-5,13-dien-1-säure versetzt man in 50 Volumenteilen Methanol mit 0,6 Gewichtsteilen p-Toluolsulfonsäure. Man rührt 5 Stunden bei 25°C, setzt 20 Volumenteile Wasser zu, neutralisiert mit Natriumhydrogencarbonat, dampft die Lösungsmittel im Vakuum ab, extrahiert den Rückstand mit Essigester, wäscht die Essigesterphase mit halbkonzentrierter wäßriger Natriumchloridlösung, trocknet und dampft im Vakuum ein. Der Rückstand wird chromatographisch an Kieselgel gereinigt. Man erhält 3,5 Gewichtsteile (5Z,9α,11α,13E,15α)-9,11,15-Trihydroxy-19-tert-butoxycarbonyl-20-nor-prosta-5,13-dien-1-säure-methylester (Ausbeute: 68% der Theorie)

IR: $\nu$=3360 und 1723 $cm^{-1}$

$^1$H-NMR ($CDCl_3$): $\delta$ = 3,66 (Singulett) und 1,46 (Singulett) ppm.

Beispiel 363

(5Z, 11α, 13E, 15α)-9-Oxo-11,15-dihydroxy-19-tert-butoxycarbonyl-20-nor-prosta-5,13-dien-1-säuremethylester

Zu 4,0 Gewichtsteilen (5Z, 11α, 13E, 15α)-9-Oxo-11,15-dihydroxy-19-tert-butoxycarbonyl-20-nor-prosta-5,13-dien-1-säure läßt man in 40 Volumenteilen Methanol und 4 Volumenteilen Wasser 20 Volumenteile einer 1,1-molaren ätherischen Diazomethanlösung einfließen. Man rührt 1 Stunde bei 25°C, zersetzt überschüssiges Diazomethan mit Essigsäure, dampft die Lösungsmittel ab, filtriert über Kieselgel mit Essigester und erhält nach dem Eindampfen des Essigesters 3,8 Gewichtsteile (5Z, 11α, 13E, 15α)-9-Oxo-11,15-dihydroxy-19-tert-butoxycarbonyl-20-nor-prosta-5,13-dien-1-säuremethylester (Ausbeute: 97% der Theorie).

$^1$H-NMR ($CDCl_3$): $\delta$ = 5,50-5,75 (Multiplett), 5,21-5,50 (Multiplett), 3,62 (Singulett) und 1,44 (Singulett) ppm.

Patentansprüche

1) Prostaglandin-Analoga der allgemeinen Formel (I)

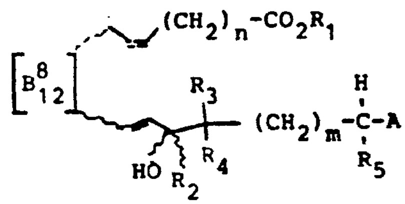

(I)

in welcher

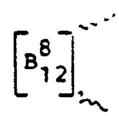 für die Teilstrukturen

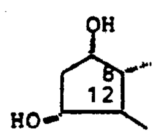

(Ia)

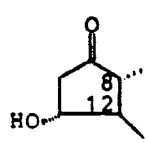

(Ib)

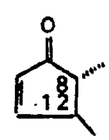

(Ic)

oder

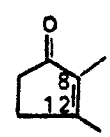

(Id)

steht.

$R_1$ für Wasserstoff oder einen gegebenenfalls substituierten Alkylrest steht,

$R_2$ für Wasserstoff oder einen Alkylrest steht,

$R_3$ und $R_4$ gleich oder verschieden sind und für Wasserstoff oder einen gegebenenfalls substituierten Alkylrest oder Aralkylrest stehen, wobei einer der Substituenten immer Wasserstoff ist, wenn der andere für Aralkyl steht,

$R_5$ für Wasserstoff oder einen gegebenenfalls substituierten Alkylrest oder Aralkylrest steht,

m für die Zahlen 0 bis 8 steht,

n für die Zahlen 2 bis 6 steht,

A für Cyano, Carboxy, Alkoxycarbonyl, N-disubstituiertes Carbamoyl steht und,

falls $R_1$ Wasserstoff bedeutet, auch deren physiologisch verträgliche Salze.

2) Verfahren zur Herstellung von Prostaglandin-Analoga gemäß Anspruch 1, dadurch gekennzeichnet, daß man

A) einen Aldehyd der allgemeinen Formel (II)

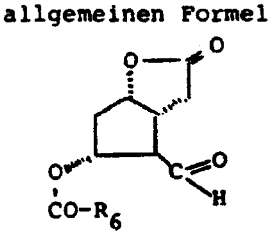

(II)

in welcher

$R_6$ für einen gegebenenfalls substituierten Alkyl- oder Arylrest steht, mit einem Phosphonester der allgemeinen Formel (III)

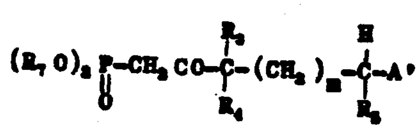

(III)

in welcher

$R_7$ für einen gegebenenfalls substituierten Alkylrest steht,

A' für Cyano, Alkoxycarbonyl oder N-disubstituiertes Carbamoyl steht und

$R_3$, $R_4$, $R_5$ und m die im Anspruch 1 angegebene Bedeutung haben,

zu einem α,ß-ungesättigten Keton der Formel (IV)

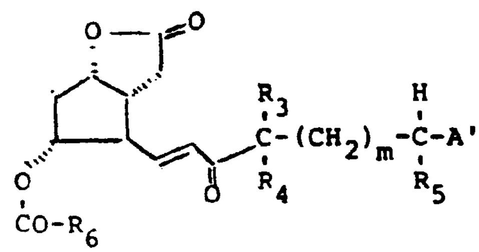

(IV)

in welcher
$R_3$, $R_4$, $R_5$, $R_6$, m und A' die oben genannte Bedeutung haben,

in Anwesenheit einer Base umsetzt und anschließend das α,β-ungesättigte Keton (IV) mit einem komplexen Metallborhydrid oder gegebenenfalls mit einer metallorganischen Alkylverbindung zu einem Allylalkohol der allgemeinen Formel (V)

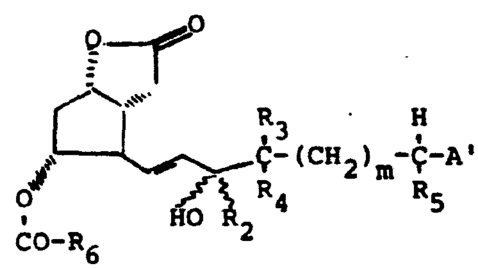

(V)

in welcher
$R_2$, $R_3$, $R_4$, $R_5$, $R_6$, m und A' die oben aufgeführte Bedeutung haben,

umsetzt, diesen durch Abspaltung der Gruppe CO-$R_6$ in ein Diol (VI) überführt, gegebenenfalls Schutzgruppen einführt und das so erhaltene Lacton der allgemeinen Formel (VI:

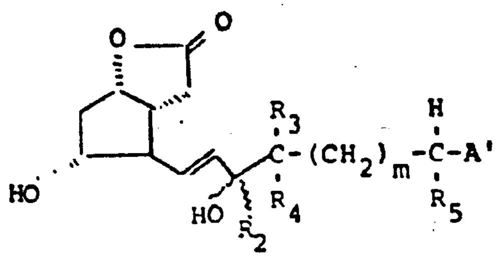 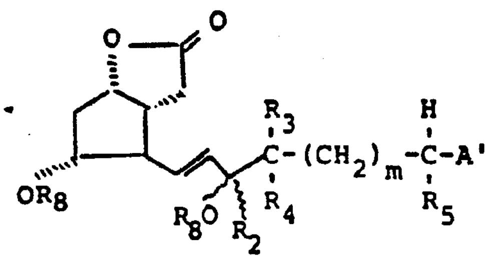

(VI) (VII)

in welcher
$R_8$ für einen Organosilyl - oder Ätherrest steht,
$R_2$, $R_3$, $R_4$, $R_5$, A' und m die obige Bedeutung haben,

zu einem Lactol der allgemeinen Formel (VIII)

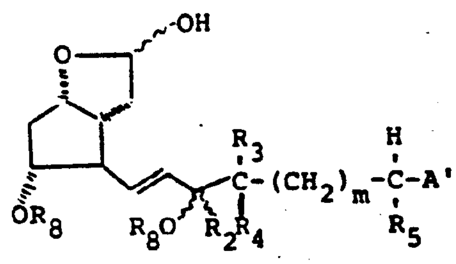

(VIII)

in welcher
$R_2$, $R_3$, $R_4$, $R_5$, $R_8$, A' und m die obige Bedeutung haben,

reduziert und das so erhaltene Lactol mit einem Phosphoniumsalz der allgemeinen Formel (IX)

$$\left[(R_9)_3{}^{\oplus}PCH_2(CH_2)_nCO_2H\right] X^{\ominus} \qquad \text{(IX)}$$

in welcher
$R_9$ für einen Arylrest,
X für ein Halogen und
n für eine Zahl zwischen 2 und 6 stehen,
in Anwesenheit einer Base zu einer Säure der allgemeinen Formel (X)

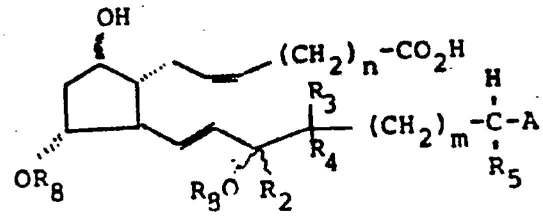

(X)

in welcher
$R_2$, $R_3$, $R_4$, $R_5$, $R_8$, A, m und n die obige Bedeutung haben,

umsetzt und anschließend für den Fall, daß in der Verbindung der allgemeinen Formel (I)

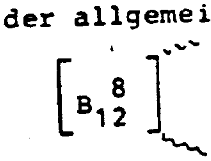 für 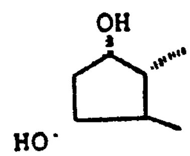 steht

mit einer Säure gemäß dem Reaktionschema I die eingeführten Schutzgruppen, gegebenenfalls unter Veresterung, wieder abspaltet und so Verbindungen mit der allgemeinen Formel (Ia) erhält,

oder

B) für den Fall, daß in der Verbindung der allgemeinen Formel (I)

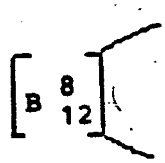 für 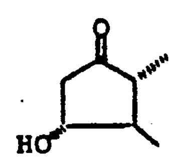 steht,

(X) zum Keton der allgemeinen Formel (XI)

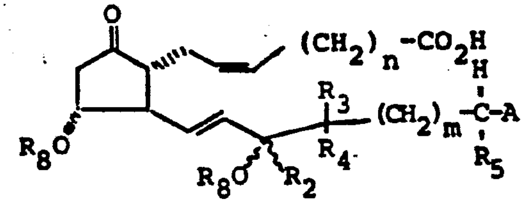

in welcher
$R_2$, $R_3$, $R_4$, $R_5$, $R_8$, A, m und n die obige Bedeutung haben,

oxidiert und anschließend die Schutzgruppen, gegebenenfalls unter Veresterung abspaltet und so Verbindungen der allgemeinen Formel (Ib) erhält,

oder für den Fall, daß in der Verbindung der allgemeinen Formel (I)

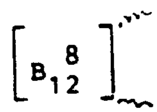 für 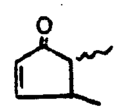 steht,

die Verbindung (Ib) oder(XI) mit einer Säure umsetzt und so die Verbindung der allgemeinen Formel (Ic) erhält,

oder für den Fall, daß in der Verbindung der allgemeinen Formel (I)

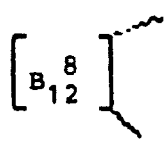 für 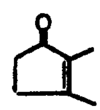 steht,

die Verbindung (Ib) mit einer Base umsetzt und so die Verbindung der allgemeinen Formel (Id) erhält.

3) Arzneimittel enthaltend mindestens eine Prostaglandin-Verbindung gemäß Anspruch 1.

4) Verfahren zur Herstellung von Arzneimittel, dadurch gekennzeichnet, daß man Prostaglandin-Analoga gemäß Anspruch 1 mit Hilfs- und Trägerstoffen vermischt.

5) Verwendung von Prostaglandinderivaten gemäß Anspruch 1 zur Beeinflussung hormonaler Reaktionen, dadurch gekennzeichnet, daß man sie im Bedarfsfall appliziert.

0000710

Europäisches Patentamt

# EUROPÄISCHER RECHERCHENBERICHT

Nummer der Anmeldung
EP 78 10 0426

| EINSCHLÄGIGE DOKUMENTE | | | KLASSIFIKATION DER ANMELDUNG (Int.Cl.²) |
|---|---|---|---|
| Kategorie | Kennzeichnung des Dokuments mit Angabe, soweit erforderlich, der maßgeblichen Teile | betrifft Anspruch | |
| P | US - A - 4 045 465 (H.J.E. HESS et al.)<br>& DE - A - 2 733 976<br>& BE - A - 857 535<br>& FR - A - 2 372 156<br>* Ansprüche 1-3; Spalten 1-4, 7,8 *<br>-- | 1-5 | C 07 C 177/00 |
| P | US - A - 4 074 044 (H.J.E. HESS et al.)<br>& DE - A - 2 733 976<br>& BE - A - 857 535<br>& FR - A - 2 372 156<br>* Ansprüche 1-3; Spalten 1,2 *<br>-- | 1-5 | RECHERCHIERTE SACHGEBIETE (Int. Cl.²)<br>C 07 C 177/00 |
| P | US - A - 4 078 021 (H.J.E. HESS et al.)<br>& DE - A - 2 733 976<br>& BE - A - 857 535<br>& FR - A . 2 372 156<br>* Spalte 2 *<br>---- | 1-5 | |

KATEGORIE DER GENANNTEN DOKUMENTE
- X: von besonderer Bedeutung
- A: technologischer Hintergrund
- O: nichtschriftliche Offenbarung
- P: Zwischenliteratur
- T: der Erfindung zugrunde liegende Theorien oder Grundsätze
- E: kollidierende Anmeldung
- D: in der Anmeldung angeführtes Dokument
- L: aus andern Gründen angeführtes Dokument
- &: Mitglied der gleichen Patentfamilie, übereinstimmendes Dokument

φ Der vorliegende Recherchenbericht wurde für alle Patentansprüche erstellt.

| Recherchenort | Abschlußdatum der Recherche | Prüfer |
|---|---|---|
| Den Haag | 02-11-1978 | BERTE |

EPA form 1503.1 06.78